# EUROPEAN PATENT APPLICATION

(11) **EP 4 434 437 A1**
(43) Date of publication of application: **25.09.2024**
(21) Application number: 22889990.2
(22) Date of filing: 02.11.2022
(51) Int. Cl.: A61B 1/045, A61B 34/10, G06T 7/00

(54) **COMPUTER PROGRAM, TRAINED MODEL GENERATION METHOD, AND INFORMATION PROCESSING DEVICE**

(30) Priority: 04.11.2021 JP 2021180519
(71) Applicant: Anaut Inc., Tokyo 105-0022 (JP)
(72) Inventor: KOBAYASHI, Nao, Tokyo 105-0022 (JP); KUMAZU, Yuta, Tokyo 105-0022 (JP); SENYA, Seigo, Tokyo 105-0022 (JP)
(74) Representative: Cabinet Beaumont
(86) International application number: PCT/JP2022/041056
(87) International publication number: WO 2023/080170

(57) **Abstract**

A computer program, a method for generating a learning model, and an information processing device are provided.

A computer program causing a computer to execute processing of acquiring an operative field image obtained by shooting an operative field of a scope-assisted surgery, and recognizing a pancreas portion in the acquired operative field image by inputting the acquired operative field image to a learning model trained to output information relevant to the pancreas portion included in the operative field image in accordance with input of the operative field image.

## Description

### [Technical Field]

The present invention relates to a computer program, a method for generating a learning model, and an information processing device.

### [Background Art]

In a laparoscope surgery, for example, a surgery for removing a diseased portion such as a malignant tumor formed in the body of a patient is performed. In this case, the inside of the patient is shot with a laparoscope, and the obtained operative field image is displayed on a monitor (for example, refer to Patent Document 1).

### [Prior Art Document]

### [Patent Document]

[Patent Document 1] Japanese Patent Laid-Open Publication No. 2005-287839

### [Summary of Invention]

### [Problems to be Solved by Invention]

In the related art, it is difficult to recognize a pancreas portion from the operative field image and report the pancreas portion to an operator.

An object of the present invention is to provide a computer program, a method for generating a learning model, and an information processing device enabling a recognition result of a pancreas portion to be output.

### [Means for Solving Problems]

A computer program in one aspect of the present invention is a computer program causing a computer to execute processing of acquiring an operative field image obtained by shooting an operative field of a scope-assisted surgery, and recognizing a pancreas portion in the acquired operative field image by inputting the acquired operative field image to a learning model trained to output information relevant to the pancreas portion included in the operative field image in accordance with input of the operative field image.

A method for generating a learning model in one aspect of the present invention causes a computer to execute processing of acquiring training data including an operative field image obtained by shooting an operative field of a scope-assisted surgery, and ground truth data indicating a pancreas portion in the operative field image, and generating a learning model for outputting information relevant to the pancreas portion in accordance with input of the operative field image on the basis of a set of acquired training data pieces.

An information processing device in one aspect of the present invention, includes an acquisition unit that acquiring an operative field image obtained by shooting an operative field of a scope-assisted surgery, a recognition unit recognizing a pancreas portion in the acquired operative field image by inputting the acquired operative field image to a learning model trained to output information relevant to the pancreas portion included in the operative field image in accordance with input of the operative field image, and an output unit outputting information based on a recognition result by the recognition unit.

### [Effects of Invention]

According to this application, the recognition result of the pancreas portion can be output.

### [Brief Description of Drawings]

FIG. 1 is a schematic view illustrating a schematic configuration of a laparoscope surgery assistance system according to Embodiment 1.
FIG. 2 is a block diagram illustrating an internal configuration of an information processing device.
FIG. 3 is a schematic view illustrating an example of an operative field image.
FIG. 4 is a schematic view illustrating a configuration example of a learning model.
FIG. 5 is a flowchart illustrating a generation procedure of a learning model.
FIG. 6 is a flowchart illustrating an execution procedure of surgery assistance.
FIG. 7 is a schematic view illustrating a display example of a recognition image according to Embodiment 1.
FIG. 8 is a schematic view illustrating a display example according to a method of the related art.
FIG. 9 is a schematic view illustrating a display example of a recognition image in Embodiment 2.
FIG. 10 is a block diagram illustrating an internal configuration of an information processing device according to Embodiment 4.
FIG. 11 is a flowchart illustrating a procedure of processing executed by the information processing device according to Embodiment 4.
FIG. 12 is an explanatory diagram illustrating an analytical method of a computation result.
FIG. 13 is a diagram illustrating an example of an evaluation coefficient table.
FIG. 14 is a diagram illustrating an example of a score computation result.
FIG. 15 is a flowchart illustrating a procedure of processing executed by an information processing device according to Embodiment 5.
FIG. 16 is a flowchart illustrating a procedure of processing executed by an information processing device according to Embodiment 6.
FIG. 17 is a schematic view illustrating a display example of a recognition result according to a confidence.
FIG. 18 is a flowchart illustrating a display switching procedure in Embodiment 7.
FIG. 19 is an explanatory diagram illustrating a display example in Embodiment 8.
FIG. 20 is a flowchart illustrating a procedure of processing executed by an information processing device according to Embodiment 8.
FIG. 21 is an explanatory diagram illustrating a first display example in Embodiment 9.
FIG. 22 is an explanatory diagram illustrating a second display example in Embodiment 9.
FIG. 23 is a schematic view illustrating a configuration example of a user interface provided in an information processing device.

### [Mode for Carrying out Invention]

Hereinafter, an aspect in which the present invention is applied to an assistance system of a laparoscope surgery will be described in detail by using the drawings. Note that, the present invention is not limited to the laparoscope surgery, and can be applied to the overall scope-assisted surgery using an imaging device, such as a thoracoscope, a gastrointestinal endoscope, a cystoscope, an arthroscope, a robot-assisted endoscope, a spinal endoscope, a surgical microscope, a neuroendoscope, and an exoscope.

### (Embodiment 1)

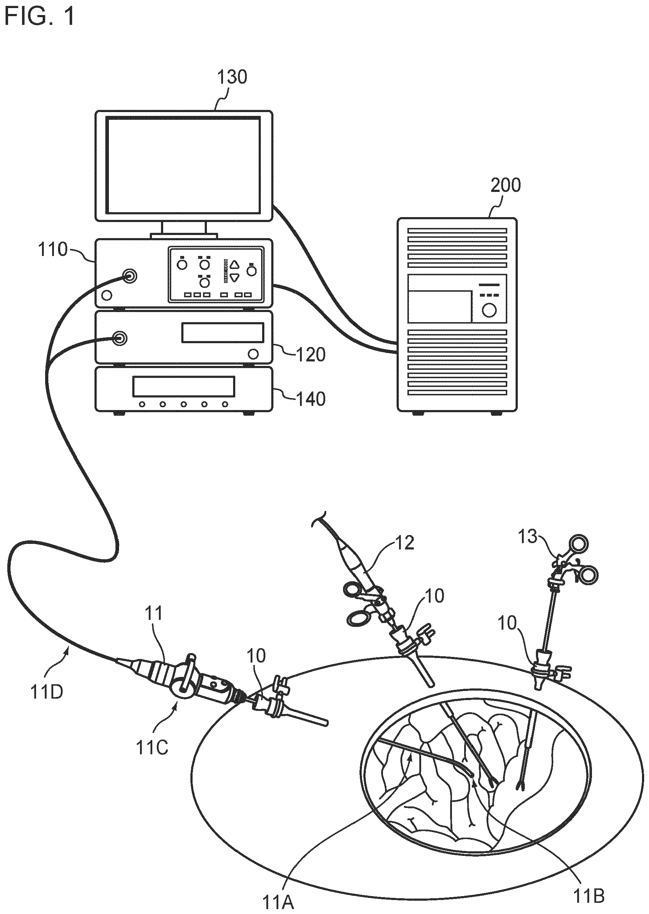

FIG. 1 is a schematic view illustrating a schematic configuration of a laparoscope surgery assistance system according to Embodiment 1. In a laparoscope surgery, a plurality of opening instruments referred to as a chest tube 10 are attached to the abdominal wall of a patient, and an instrument such as a laparoscope 11, an energy treatment tool 12, or forceps 13 is inserted into the body of the patient from the opening provided in the chest tube 10, instead of performing a laparotomy. An operator performs a treatment such as excising an affected area by using the energy treatment tool 12 while observing an image (an operative field image) of the inside of the body of the patient, which is shot by the laparoscope 11, in real-time. A surgical tool such as the laparoscope 11, the energy treatment tool 12, or the forceps 13 is retained by the operator, a robot, or the like. The operator is a healthcare worker involved in the laparoscope surgery, and includes an operator, an assistant, a nurse, a medical doctor monitoring a surgery, and the like.

The laparoscope 11 includes an insertion portion 11A inserted into the body of the patient, an imaging device 11B built in the front edge portion of the insertion portion 11A, a manipulation unit 11C provided in the tail edge portion of the insertion portion 11A, and a universal cord 11D for the connection to a camera control unit (CCU) 110 or a light source device 120.

The insertion portion 11A of the laparoscope 11 includes a rigid tube. A bending portion is provided in the front edge portion of the rigid tube. A bending mechanism of the bending portion is a known mechanism incorporated in a general laparoscope, and is configured to bend, for example, in four directions of up, down, left, and right, by pulling a manipulation wire in conjunction with the manipulation of the manipulation unit 11C. Note that, the laparoscope 11 is not limited to a flexible scope including the bending portion as described above, and may be a rigid scope not including the bending portion, or may be an imaging device not including the bending portion or the rigid tube. Further, the laparoscope 11 may be a 360-degree camera imaging a range of 360 degrees.

The imaging device 11B includes a solid state image sensor such as a complementary metal oxide semiconductor (CMOS), and a driver circuit including a timing generator (TG), an analog signal processing circuit (AFE), and the like. The driver circuit of the imaging device 11B imports signals of each color of RGB output from the solid state image sensor, in synchronization with a clock signal output from TG, performs required processing such as filter-out, amplification, and AD conversion, in AFE, and generates image data in a digital format. The driver circuit of the imaging device 11B transmits the generated image data to the CCU 110 through the universal cord 11D.

The manipulation unit 11C includes an angular lever, a remote switch, or the like, which is manipulated by the operator. The angular lever is a manipulation tool receiving a manipulation for bending the bending portion. Instead of the angular lever, a bending manipulation knob, a joystick, and the like may be provided. The remote switch, for example, includes a switch switching an observation image to moving image display or still image display, a zoom switch zooming in or out the observation image, and the like. A specific function set in advance may be allocated to the remote switch, or a function set by the operator may be allocated to the remote switch.

In addition, an oscillator including a linear resonant actuator, a piezoelectric actuator, or the like may be built in the manipulation unit 11C. In a case where an event to be reported to the operator manipulating the laparoscope 11 occurs, the CCU 110 may oscillate the manipulation unit 11C by operating the oscillator built in the manipulation unit 11C to report the occurrence of the event to the operator.

Inside the insertion portion 11A, the manipulation unit 11C, and the universal cord 11D of the laparoscope 11, a transmission cable for transmitting a control signal output from the CCU 110 to the imaging device 11B, or the image data output from the imaging device 11B, a light guide guiding illumination light exiting from the light source device 120 to the front edge portion of the insertion portion 11A, and the like are arranged. The illumination light exiting from the light source device 120 is guided to the front edge portion of the insertion portion 11A through the light guide, and is applied to an operative field through an illumination lens provided in the front edge portion of the insertion portion 11A. Note that, in this embodiment, the light source device 120 is described as an independent device, but the light source device 120 may be built in the CCU 110.

The CCU 110 includes a control circuit controlling the operation of the imaging device 11B provided in the laparoscope 11, an image processing circuit processing the image data from the imaging device 11B, which is input through the universal cord 11D, and the like. The control circuit includes a central processing unit (CPU), a read only memory (ROM), a random access memory (RAM), and the like, and outputs the control signal to the imaging device 11B and controls shooting start, shooting stop, zooming, or the like, in accordance with the manipulation of various switches provided in the CCU 110 or the manipulation of the manipulation unit 11C provided in the laparoscope 11. The control circuit is not limited to the CPU, the ROM, and the RAM, and may include a graphics processing unit (GPU), a field programmable gate array (FPGA), or the like. The image processing circuit includes a digital signal processor (DSP), an image memory, and the like, and performs suitable processing such as color separation, color interpolation, gain correction, white balance adjustment, and gamma correction with respect to the image data input through the universal cord 11D. The CCU 110 generates a frame image for a moving image from the processed image data, and sequentially outputs each of the generated frame images to an information processing device 200 described below. The frame rate of the frame image, for example, is 30 frames per second (FPS).

The CCU 110 may generate video data based on a predetermined standard such as a national television system committee (NTSC), a phase alternating line (PAL), and digital imaging and communication in medicine (DICOM). By the CCU 110 outputting the generated video data to a display device 130, it is possible to display the operative field image (video) on a display screen of the display device 130 in real-time. The display device 130 is a monitor including a liquid crystal panel, an organic electro-luminescence (EL) panel, or the like. In addition, the CCU 110 may output the generated video data to a video recording device 140 such that the video data is recorded in the video recording device 140. The video recording device 140 includes a recording device such as a hard disk drive (HDD) recording the video data output from the CCU 110, together with an identifier for identifying each surgery, a surgery date and time, a surgical site, a patient name, an operator name, and the like.

The information processing device 200 generates assistance information relevant to a laparoscope surgery, on the basis of the image data input from the CCU 110 (that is, image data of the operative field image obtained by shooting the operative field). Specifically, the information processing device 200 performs processing of recognizing a net pancreas portion excluding a portion corresponding to a blood vessel, fat, an interlobular groove, or an interlobular shadow appearing on the surface of the pancreas from a region set by the outer edge of the pancreas included in the operative field image, and superimposing the pancreas portion recognized by excluding the portion on the operative field image to display the pancreas portion on the display device 130. Here, the pancreas portion recognized by the information processing device 200 is represented by a set of pixels corresponding to the pancreas in the operative field image. Note that, the portion corresponding to the blood vessel, the fat, the interlobular groove, or the interlobular shadow appearing on the surface of the pancreas may be any one portion, or two or more portions, among the blood vessel, the fat, the interlobular groove, and the interlobular shadow.

In this embodiment, a configuration will be described in which the recognition processing of the pancreas portion is executed in the information processing device 200, but a function equivalent to that of the information processing device 200 may be provided in the CCU 110, and the recognition processing of the pancreas portion may be executed in the CCU 110.

Hereinafter, the internal configuration of the information processing device 200, and the recognition processing and display processing executed by the information processing device 200 will be described.

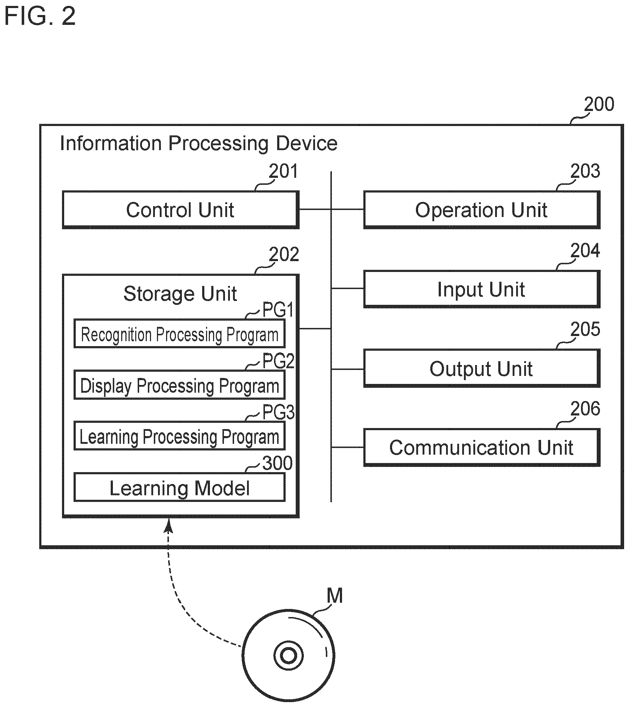

FIG. 2 is a block diagram illustrating the internal configuration of the information processing device 200. The information processing device 200 is a dedicated or general-purpose computer including a control unit 201, a storage unit 202, a operation unit 203, an input unit 204, an output unit 205, a communication unit 206, and the like. The information processing device 200 may be a computer provided inside a surgery room, or may be a computer provided outside the surgery room. In addition, the information processing device 200 may be a server provided inside the hospital in which the laparoscope surgery is performed, or may be a server provided outside the hospital. The information processing device 200 may be used to assist a remote surgery.

The control unit 201, for example, includes a CPU, a ROM, a RAM, and the like. In the ROM provided in the control unit 201, a control program for controlling the operation of each hardware unit provided in the information processing device 200, and the like are stored. The CPU in the control unit 201 executes the control program stored in the ROM or various computer programs stored in the storage unit 202 described below, and controls the operation of each hardware unit to allow the entire device to function as the information processing device in this application. In the RAM provided in the control unit 201, data or the like used during the execution of the computation is transitorily stored.

In this embodiment, the control unit 201 includes the CPU, the ROM, and the RAM, but the configuration of the control unit 201 is any configuration, and for example, may be a computation circuit or a control circuit including one or a plurality of GPUs, FPGAs, quantum processors, volatile or non-volatile memories, and the like. In addition, the control unit 201 may have a function as a clock outputting date and time information, a timer measuring the elapsed time from when a measurement start instruction is applied to when a measurement end instruction is applied, a counter counting the number, or the like.

The storage unit 202 includes a storage device using a hard disk, a flash memory, or the like. In the storage unit 202, the computer program executed by the control unit 201, various data pieces acquired from the outside, various data pieces generated inside the device, and the like are stored.

The computer program stored in the storage unit 202 includes a recognition processing program PG1 for allowing the control unit 201 to execute the recognition processing of the pancreas portion included in the operative field image, a display processing program PG2 for allowing the control unit 201 to execute processing of displaying assistance information based on a recognition result on the display device 130, and a learning processing program PG3 for generating a learning model 300. Note that, the recognition processing program PG1 and the display processing program PG2 are not necessarily an independent computer program, and may be implemented as one computer program. Such programs, for example, are provided by a non-transitory recording medium M in which the computer program is readably recorded. The recording medium M is a portable memory such as a CD-ROM, a USB memory, and a secure digital (SD) card. The control unit 201 reads out a desired computer program from the recording medium M by using a reader that is not illustrated, and stores the read computer program in the storage unit 202. Alternatively, the computer program described above may be provided by communication using the communication unit 206.

In addition, in the storage unit 202, the learning model 300 used in the recognition processing program PG1 described above is stored. The learning model 300 is a learning model trained to output information relevant to the pancreas portion included in the operative field image, in response to the input of the operative field image. The learning model 300 is described by definition information thereof. The definition information of the learning model 300 includes information of layers provided in the learning model 300, information of nodes configuring each of the layers, and parameters such as weights and biases between the nodes. Such parameters are trained by executing a predetermined learning algorithm using training data including the operative field image obtained by shooting the operative field, and ground truth data indicating the pancreas portion in the operative field image. The configuration and the generation procedure of the learning model 300 will be described below in detail.

The operation unit 203 includes a input device such as a keyboard, a mouse, a touch panel, a non-contact panel, a stylus pen, and voice input using a microphone. The operation unit 203 receives the manipulation of the operator or the like, and outputs information relevant to the received manipulation to the control unit 201. The control unit 201 executes suitable processing, in accordance with the manipulation information input from the operation unit 203. Note that, in this embodiment, the information processing device 200 includes the operation unit 203, but the manipulation may be received through various devices such as the CCU 110 connected to the outside.

The input unit 204 includes a connection interface for connecting an input device. In this embodiment, the input device connected to the input unit 204 is the CCU 110. The image data of the operative field image that is shot by the laparoscope 11 and processed by the CCU 110 is input to the input unit 204. The input unit 204 outputs the input image data to the control unit 201. In addition, the control unit 201 may store the image data acquired from the input unit 204 in the storage unit 202.

The output unit 205 includes a connection interface for connecting an output device. In this embodiment, the output device connected to the output unit 205 is the display device 130. In a case where information to be reported to the operator or the like, such as a recognition result by the learning model 300, is generated, the control unit 201 outputs the generated information to the display device 130 by the output unit 205 to display the information on the display device 130. In this embodiment, the display device 130 is connected to the output unit 205 as the output device, but an output device such as a speaker outputting a sound may be connected to the output unit 205.

The communication unit 206 includes a communication interface for transmitting and receiving various data pieces. The communication interface provided in the communication unit 206 is a communication interface based on a wired or wireless communication standard that is used in Ethernet (Registered Trademark) or WiFi (Registered Trademark). In a case where data to be transmitted is input from the control unit 201, the communication unit 206 transmits the data to be transmitted to a designated destination. In addition, in a case where data transmitted from an external device is received, the communication unit 206 outputs the received data to the control unit 201.

The information processing device 200 is not limited to a single computer, and may be a computer system including a plurality of computers or peripheral devices. The information processing device 200 may be a virtual machine that is virtually constructed by software.

Next, the operative field image input to the information processing device 200 will be described.

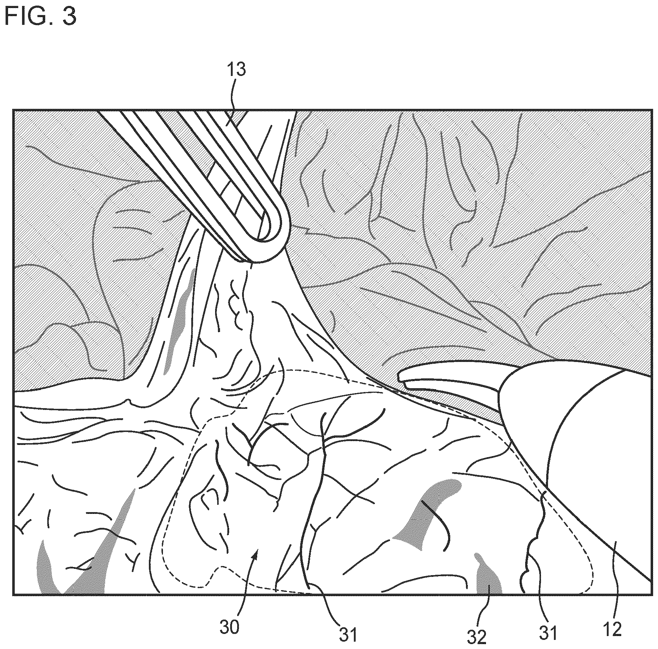

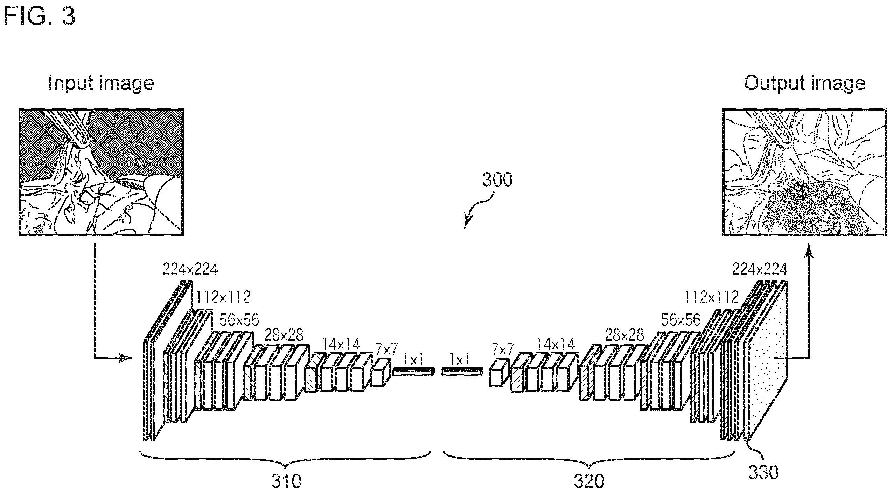

FIG. 3 is a schematic view illustrating an example of the operative field image. The operative field image in this embodiment is an image obtained by shooting the inside of the abdominal cavity of the patient with the laparoscope 11. The operative field image is not necessarily a raw image output from the imaging device 11B of the laparoscope 11, and may be an image (a frame image) processed by the CCU 110 or the like. In addition, the operative field image may be an image output to the display device 130 from the CCU 110, or may be an image processed by an image processing device (not illustrated) detachably mounted on the laparoscope 11. Further, the operative field image may be a recorded video that has been recorded in the video recording device 140.

The operative field image illustrated in FIG. 3 as an example represents a scene in which a membrane covering the pancreas 30 is pulled with the forceps 13 to peel off the membrane or a connective tissue with the energy treatment tool 12. Such am operative field image includes not only various organs including the pancreas 30, membranes covering the various organs, and connective tissues existing between the organs, but also blood vessels 31, fat 32, and the like appearing on the surface of the organs. In FIG. 3, the outer edge of the pancreas 30 is indicated by a broken line. The operative field image may include, in addition to the tissues described above, tissues of a diseased portion such as a tumor, fat existing between the tissues, and the like. The operator advances the peeling of the membrane or the connective tissue with an instrument such as the forceps 13 or the energy treatment tool 12 while grasping a positional relationship (an anatomical structure) between the tissues to peel off the tissue including the diseased portion.

In order for the operator to peel off the membrane covering the pancreas or the connective tissue connecting the tissues or peel off the tissue including the diseased portion, it is necessary to clearly grasp the position of the pancreas. However, in general, the pancreas is adjacent to other organs such as the stomach, the duodenum, and the spleen, and it is difficult to specify the boundary between the pancreas and the other organs. In a case where a region including the pancreas is recognized and the entire recognized region is presented with a color by a method of the related art using artificial intelligence, it is easy to specify the boundary between the pancreas and the other organs. However, in a case where the entire recognized region is presented with a color, the blood vessel, the fat, the interlobular groove, or the shadow appearing on the surface of the pancreas are also hidden, and thus, information required for the operator performing the surgery may be lost.

Therefore, the information processing device 200 according to this embodiment performs surgery assistance with respect to the operator by recognizing the net pancreas portion excluding the portion corresponding to the blood vessel, the fat, the interlobular groove, or the interlobular shadow appearing on the surface of the pancreas from the region (a pancreas region) surrounded by the outer edge of the pancreas using the learning model 300, and displaying the pancreas portion recognized by excluding the portion on the operative field image as a recognition image.

Hereinafter, the learning model 300 will be described.

FIG. 4 is a schematic view illustrating a configuration example of the learning model 300. The learning model 300 is a learning model for performing image segmentation, and for example, is constructed by a neural network including a convolutional layer such as SegNet. The learning model 300 is not limited to SegNet, and may be constructed by using any neural network capable of performing image segmentation, such as a fully convolutional network (FCN), a U-shaped network (U-Net), and a pyramid scene parsing network (PSPNet). In addition, the learning model 300 may be constructed by using a neural network for object detection such as you only look once (YOLO) and a single shot multi-box detector (SSD), instead of the neural network for image segmentation.

In this embodiment, the input image to the learning model 300 is the operative field image obtained from the laparoscope 11. The learning model 300 is trained to output the information relevant to the pancreas portion included in the operative field image, in response to the input of the operative field image.

The learning model 300, for example, includes an encoder 310, a decoder 320, and a softmax layer 330. The encoder 310 is configured by alternately arranging a convolutional layer and a pooling layer. The convolutional layer is multilayered into two to three layers. In the example of FIG. 4, the convolutional layer is illustrated without hatching, and the pooling layer is illustrated with hatching.

In the convolutional layer, convolutional computation between data to be input and filters each having a predetermined size (for example, 3 × 3, 5 × 5, or the like) is performed. That is, an input value input to a position corresponding to each element of the filter, and weight coefficients set in advance in the filter are multiplied together for each element, a linear sum of multiplication values for each element is computed. By adding set biases to the computed linear sum, the output of the convolutional layer is obtained. Note that, the result of the convolutional computation may be converted by an activating function. As the activating function, for example, a rectified linear unit (ReLU) can be used. The output of the convolutional layer represents a feature map in which the features of the input data are extracted.

In the pooling layer, the amount of local statistics of the feature map output from the convolutional layer that is a higher layer connected to the input side is computed. Specifically, a window having a predetermined size (for example, 2 × 2, 3 × 3) corresponding to the position of the higher layer is set, and the amount of local statistics is computed from an input value in the window. As the amount of statistics, for example, the maximum value can be adopted. The size of a feature map output from the pooling layer is reduced (downsampled) in accordance with the size of the window. The example of FIG. 4 illustrates that an input image of 224 pixels × 224 pixels is sequentially downsampled to feature maps of 112 × 112, 56 × 56, 28 × 28, ..., 1 × 1 by sequentially repeating the computation of the convolutional layer and the computation of the pooling layer in the encoder 310.

The output (in the example of FIG. 4, the feature map of 1 × 1) of the encoder 310 is input to the decoder 320. The decoder 320 is configured by alternately arranging a deconvolutional layer and a depooling layer. The deconvolutional layer is multilayered into two to three layers. In the example of FIG. 4, the deconvolutional layer is illustrated without hatching, and the depooling layer is illustrated with hatching.

In the deconvolutional layer, deconvolutional computation is performed with respect to the input feature map. The deconvolutional computation is computation for restoring the feature map before the convolutional computation, under presumption that the input feature map is the result of the convolutional computation using a specific filter. In such computation, when the specific filter is represented in a matrix, a product between a transposed matrix with respect to the matrix and the input feature map is computed to generate a feature map for output. Note that, a computation result of the deconvolutional layer may be converted by the activating function such as ReLU.

The depooling layers provided in the decoder 320 are individually associated with the pooling layers provided in the encoder 310 on a one-to-one basis, and the associated pairs have substantially the same size. The depooling layer enlarges (upsamples) again the size of the feature map downsampled in the pooling layer of the encoder 310. The example of FIG. 4 illustrates that sequential upsampling to feature maps of 1 × 1, 7 × 7, 14 × 14, ..., 224 × 224 is performed by sequentially repeating the computation of the convolutional layer and the computation of the pooling layer in the decoder 320.

The output (in the example of FIG. 4, the feature map of 224 × 224) of the decoder 320 is input to the softmax layer 330. The softmax layer 330 applies a softmax function to the input value from the deconvolutional layer connected to the input side to output the probability of a label for identifying a portion at each position (pixel). In this embodiment, a label for identifying the pancreas portion may be set, and whether to correspond to the pancreas portion may be identified in pixel unit. By extracting a pixel in which the probability of the label output from the softmax layer 330 is a threshold value or more (for example, 70% or more), an image (hereinafter, referred to as a recognition image) indicating the recognition result of the pancreas portion is obtained.

Note that, in the example of FIG. 4, the image of 224 pixels × 224 pixels is the input image to the learning model 300, but the size of the input image is not limited to the above, and can be suitably set in accordance with the processing capability of the information processing device 200, the size of the operative field image obtained from the laparoscope 11, and the like. In addition, the input image to the learning model 300 is not necessarily the entire operative field image obtained from the laparoscope 11, and may be a partial image generated by cutting out an attention region of the operative field image. Since the attention region including a treatment target is often positioned in the vicinity of the center of the operative field image, for example, a partial image obtained by cutting out the vicinity of the center of the operative field image into a rectangular shape such that the image is approximately half the original size may be used. By decreasing the size of the image input to the learning model 300, it is possible to improve a recognition accuracy while increasing a processing rate.

In this embodiment, in order to recognize the pancreas portion included in the operative field image to be distinguished from the portion corresponding to the blood vessel, the fat, the interlobular groove, or the interlobular shadow appearing on the surface of the pancreas, the learning model 300 for recognizing whether to corresponding to the pancreas portion in pixel unit is generated. As a preparatory stage of generating the learning model 300, annotation is implemented on the shot operative field image.

In the preparatory stage of generating the learning model 300, an operator (a specialist such as a medical doctor) performs annotation by displaying the operative field image that is video-recorded in the video recording device 140 on the display device 130, and designating a pixel corresponding to the pancreas portion in pixel unit with a mouse, a stylus pen, or the like provided as the operation unit 203. In this case, it is preferable that the operator designates the pixel corresponding to the net pancreas portion in pixel unit by excluding the portion such as the blood vessel appearing on the surface of the pancreas. A set of a plurality of operative field images used in the annotation and data (ground truth data) indicating the position of the pixel corresponding to the pancreas portion designated in each of the operative field images is stored in the storage unit 202 of the information processing device 200 as the training data for generating the learning model 300. In order to increase the number of training data pieces, a set of the operative field image generated by applying perspective transformation, reflection processing, or the like, and the ground truth data with respect to the operative field image may be included in the training data. Further, in a case where training progresses, a set of the operative field image and the recognition result (the ground truth data) of the learning model 300 obtained by inputting the operative field image may be included in the training data.

Further, when performing the annotation, the operator may label a pixel corresponding to the portion such as the blood vessel to be excluded as non-ground truth data. A set of the operative field images used in the annotation, the data (the ground truth data) indicating the position of the pixel corresponding to the pancreas designated in each of the operative field images, and the data (the non-ground truth data) indicating the position of the pixel corresponding to the designated blood vessel, fat, interlobular groove, or interlobular shadow may be stored in the storage unit 202 of the information processing device 200 as the training data for generating the learning model 300.

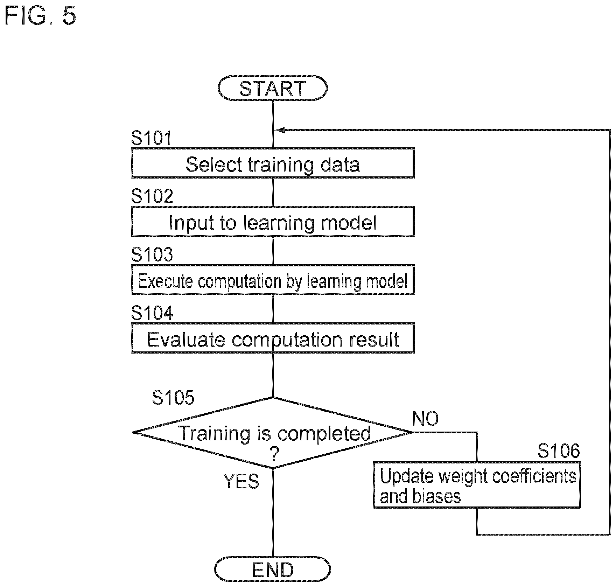

The information processing device 200 generates the learning model 300 by using the training data described above. FIG. 5 is a flowchart illustrating the generation procedure of the learning model 300. The control unit 201 of the information processing device 200 reads out the learning processing program PG3 from the storage unit 202, and executes the following procedure to generate the learning model 300. Note that, it is assumed that an initial value is applied to the definition information for describing the learning model 300, in a stage before starting training.

The control unit 201 accesses the storage unit 202 and selects a set of training data pieces from the training data prepared in advance to generate the learning model 300 (step S101). The control unit 201 inputs the operative field image included in the selected training data to the learning model 300 (step S 102), and executes the computation by the learning model 300 (step S103). That is, the control unit 201 generates the feature map from the input operative field image, and executes the computation by the encoder 310 sequentially downsampling the generated feature map, the computation by the decoder 320 sequentially upsampling the feature map input from the encoder 310, and the computation by the softmax layer 330 identifying each of the pixels of the feature map finally obtained by the decoder 320.

The control unit 201 acquires a computation result from the learning model 300, and evaluates the acquired computation result (step S104). For example, the control unit 201 may compute the degree of similarity between the image data of the pancreas portion obtained as the computation result and the ground truth data included in the training data to evaluate the computation result. The degree of similarity, for example, is computed by a Jaccard coefficient. When the pancreas portion extracted by the learning model 300 is set as A, and the pancreas portion included in the ground truth data is set as B, the Jaccard coefficient is applied by A∩B/A ∪ B × 100(%). Instead of the Jaccard coefficient, a Dice coefficient or a Simpson coefficient may be computed, or the degree of similarity may be computed by using other existing methods. In a case where the non-ground truth data is included in the training data, the control unit 201 may advance the training, with reference to the non-ground truth data. For example, in a case where the pancreas portion extracted by the learning model 300 corresponds to the other portion such as the blood vessel included in the non-ground truth data, the control unit 201 may perform processing of subtracting the degree of similarity.

The control unit 201 determines whether the training is completed, on the basis of the evaluation of the computation result (step S 105). In a case where the degree of similarity is a threshold value or more, which is set in advance, the control unit 201 is capable of determining that the training is completed.

In a case where it is determined that the training is not completed (S105: NO), the control unit 201 sequentially updates weight coefficients and biases in each of the layers of the learning model 300 from the output side toward the input side of the learning model 300, by using an error back-propagation method (step S106). The control unit 201 updates the weight coefficients and the biases of each of the layers, and then, returns the processing to step S101, and executes again the processing from step S101 to step S105.

In a case where it is determined that the training is completed in step S105 (S105: YES), the learning model 300 that has been trained is obtained, and thus, the control unit 201 ends the processing according to this flowchart.

In this embodiment, the learning model 300 is generated in the information processing device 200, but the learning model 300 may be generated by using an external computer such as a server device. In this case, the information processing device 200 may acquire the learning model 300 generated by the external computer using means such as communication, and store the acquired learning model 300 in the storage unit 202.

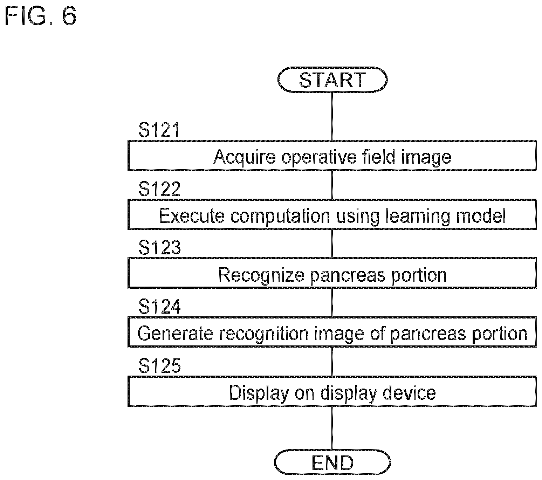

The information processing device 200 performs the surgery assistance in an operation phase after the learning model 300 is generated. FIG. 6 is a flowchart illustrating an execution procedure of the surgery assistance. The control unit 201 of the information processing device 200 reads out the recognition processing program PG1 and the display processing program PG2 from the storage unit 202, and executes the programs to execute the following procedure. In a case where the laparoscope surgery is started, the operative field image obtained by shooting the operative field with the imaging device 11B of the laparoscope 11 is continually output to the CCU 110 through the universal cord 11D. The control unit 201 of the information processing device 200 acquires the operative field image output from the CCU 110 by the input unit 204 (step S121). The control unit 201 executes the following processing each time when the operative field image is acquired.

The control unit 201 inputs the acquired operative field image to the learning model 300, and executes computation using the learning model 300 (step S122) to recognize the pancreas portion included in the operative field image (step S123). That is, the control unit 201 generates the feature map from the input operative field image, and executes the computation by the encoder 310 sequentially downsampling the generated feature map, the computation by the decoder 320 sequentially upsampling the feature map input from the encoder 310, and the computation by the softmax layer 330 identifying each of the pixels of the feature map finally obtained by the decoder 320. In addition, the control unit 201 recognizes the pixel in which the probability of the label output from the softmax layer 330 is the threshold value or more (for example, 70% or more) as the pancreas portion.

When generating the learning model 300, the annotation may be performed to recognize the pancreas portion that is in the central visual field of the operator, and in step S123, the pancreas portion existing in the central visual field of the operator may be recognized. In addition, the annotation may be performed to recognize the pancreas portion that is not in the central visual field of the operator, and in step S 123, the pancreas portion that is not in the central visual field of the operator may be recognized. Further, the annotation may be performed to recognize the pancreas portion that starts to be exposed to the operative field, and pull or excise the membrane or the layer covering the pancreas to recognize the pancreas portion in a stage where the pancreas starts to be exposed.

The control unit 201 generates the recognition image of the pancreas portion to discriminably display the pancreas portion recognized by using the learning model 300 (step S124). The generated recognition image has the same size as that of the operative field image, and is an image in which a specific color is allocated to the pixel recognized as the pancreas portion. The color allocated to the pancreas portion is arbitrarily set. For example, the color allocated to the pancreas portion may be a white color, or may be a blue color that does not exist inside the human body. In addition, information indicating a transmission rate is added to each of the pixels configuring the recognition image. For example, a non-transmissive value is set for the pixel recognized as the pancreas portion, and a transmissive value is set for the other pixel (that is, a pixel belonging to a region outside the outer edge of the pancreas portion, and a pixel belonging to the portion corresponding to the blood vessel, the fat, the interlobular groove, or the interlobular shadow).

The control unit 201 outputs the operative field image acquired in step S121 and the recognition image of the pancreas portion generated in step S124 to the display device 130 by the output unit 205, and displays the recognition image to be superimposed on the operative field image (step S125). Accordingly, the pancreas portion recognized by using the learning model 300 is displayed on the operative field image as a structure with a specific color.

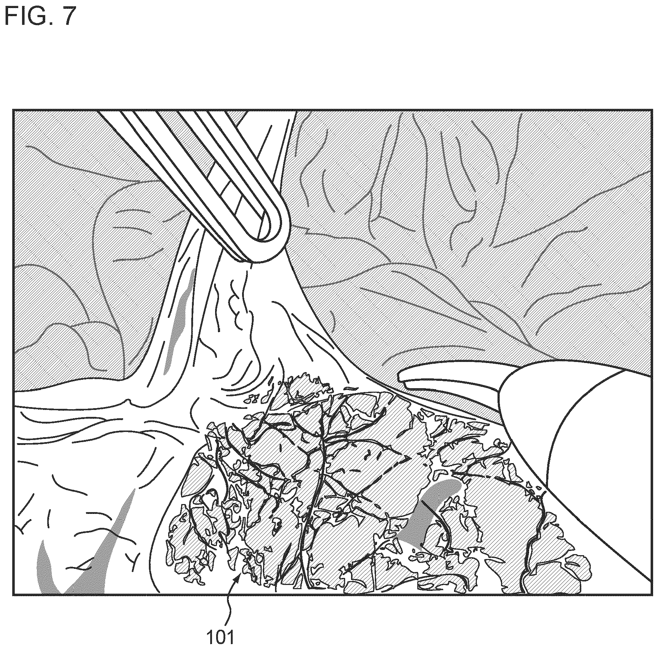

FIG. 7 is a schematic view illustrating a display example of a recognition image according to Embodiment 1. For convenience of preparing the drawing, in the display example of FIG. 7, a pancreas portion 101 recognized by using the learning model 300 is illustrated as a region with hatching. In practice, since the pancreas portion 101 is painted with a specific color such as a white color or a blue color in pixel unit, the operator is capable of recognizing the pancreas portion 101 to be distinguished from the portion such as the blood vessel by browsing the display screen.

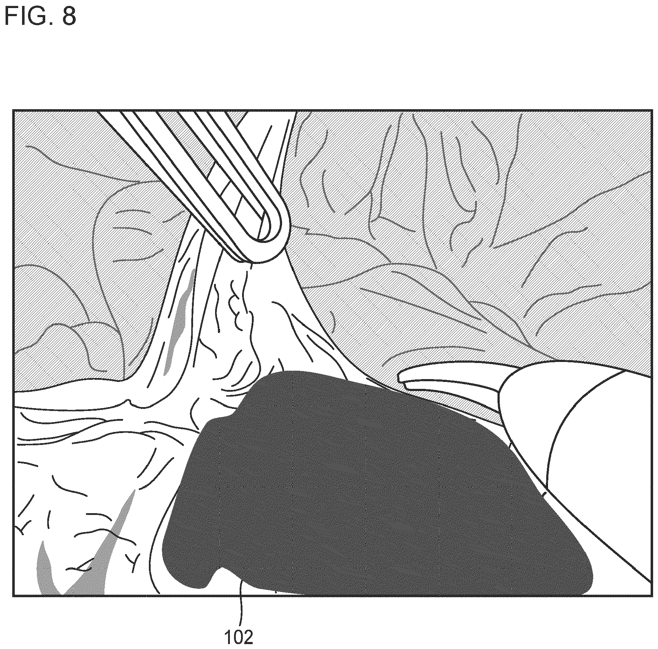

FIG. 8 is a schematic view illustrating a display example according to the method of the related art. In the method of the related art, the region including the pancreas is recognized by artificial intelligence, and the entire recognized region 102 is colored and displayed to be superimposed on the operative field image. In this case, it is difficult to visually recognize the portion corresponding to the blood vessel, the fat, the interlobular groove, or the interlobular shadow appearing on the surface of the pancreas, and there is a possibility that the information required for the operator performing the surgery may be lost.

In contrast, in the recognition method according to this embodiment, the net pancreas portion excluding the portion corresponding to the blood vessel, the fat, the interlobular groove, or the interlobular shadow from the pancreas region is recognized, and as illustrated in FIG. 7, the recognition image of the pancreas portion recognized by excluding the portion such as the blood vessel is displayed to be superimposed on the operative field image. Accordingly, it is possible to present the position of the outer edge of the pancreas (the boundary between the pancreas and the other organs) to the operator without losing the information of various tissues appearing on the surface of the pancreas.

In this embodiment, in the case of recognizing the pancreas portion included in the operative field image, the recognition image of the pancreas portion is displayed to be superimposed on the operative field image, but the superimposed display may be performed only in a case where a display instruction is applied. The display instruction may be applied by the operation unit 203 of the information processing device 200, or may be applied by the manipulation unit 11C of the laparoscope 11. In addition, the display instruction may be applied by a foot switch or the like, which is not illustrated.

In addition, in this embodiment, the recognition image of the pancreas portion is displayed to be superimposed on the operative field image, but the detection of the pancreas portion may be reported to the operator by a sound or a voice.

Further, the control unit 201 of the information processing device 200 may generate a control signal for controlling a medical device such as the energy treatment tool 12 or a surgical robot (not illustrated), on the basis of the recognition result of the pancreas portion, and output the generated control signal to the medical device.

As described above, in this embodiment, the pancreas portion can be recognized by using the learning model 300, and the recognized pancreas portion can be discriminably displayed in pixel unit, and thus, it is possible to perform visual assistance in the laparoscope surgery.

Note that, the image generated from the information processing device 200 may be used not only for the surgery assistance but also for education assistance for a doctor-in-training or the like, or may be used for the evaluation of the laparoscope surgery. For example, whether a pulling manipulation or a peeling manipulation in the laparoscope surgery is suitable is determined by comparing the image video-recorded in the video recording device 140 during the surgery with the image generated from the information processing device 200, and thus, the laparoscope surgery can be evaluated.

### (Embodiment 2)

In Embodiment 2, a configuration will be described in which a display mode is changed in accordance with a confidence when recognizing the pancreas.

Note that, the entire configuration of the laparoscope surgery assistance system, and the internal configuration of the information processing device 200, and the like are the same as those in Embodiment 1, and thus, the description thereof will be omitted.

The information processing device 200 according to Embodiment 1 described above refers to the probability output from the softmax layer 330 of the learning model 300 to allocate a specific color (for example, a white color) and a transmission rate 1 (non-transmissive) to a pixel of which the probability is the threshold value or more (for example, 50% or more), and allocates a transmission rate 0 (fully transmissive) to a pixel of which the probability is less than the threshold value, and thus, generates the recognition image indicating the pancreas portion. The information processing device 200 outputs such a recognition image, and displays the recognition image to be superimposed on the operative field image, and thus, it is possible to uniformly display (overlay display) the pancreas portion.

In contrast, the information processing device 200 according to Embodiment 2 sets a specific color (for example, a white color) for each of the pixels of the recognition image, and sets a transmission rate for each of the pixels, in accordance with the probability (the confidence) output from the softmax layer 330 of the learning model 300 to generate the recognition image of the pancreas portion. Specifically, the information processing device 200 sets the transmission rate of each of the pixels such that the transmission rate decreases as the confidence increases, and the transmission rate increases as the confidence decreases. For example, the transmission rate when the confidence is X% can be set as X/100. The information processing device 200 outputs the generated recognition image, and displays the recognition image to be superimposed on the operative field image, and thus, it is possible to attain display (software map display) according to the confidence.

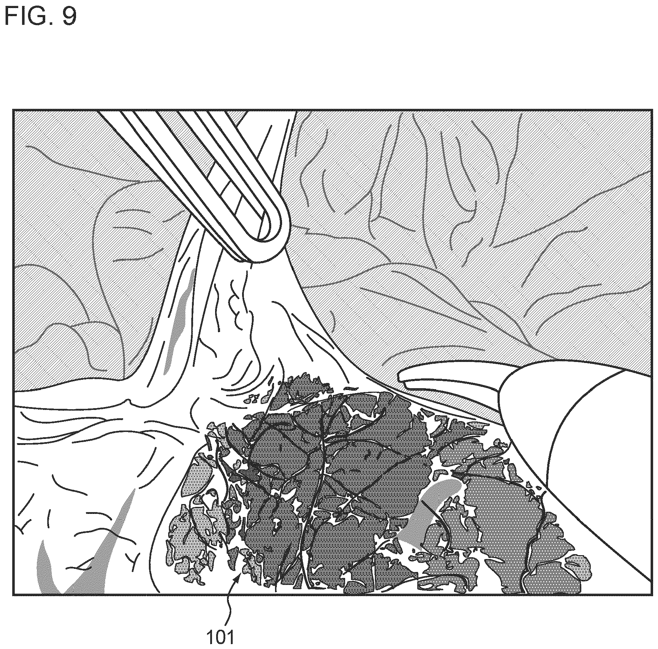

FIG. 9 is a schematic view illustrating a display example of the recognition image in Embodiment 2. For convenience of preparing the drawing, in the example of FIG. 9, the level of the transmission rate is indicated by the level of the concentration. That is, in the example of FIG. 9, the recognition image is represented such that the concentration of the pancreas portion with a high confidence is high, and the concentration of the pancreas portion with a low confidence is low.

In Embodiment 2, since it is possible to clearly display the pancreas portion with a relatively high confidence, it is possible to accurately present information useful when performing the pulling manipulation, the peeling manipulation, or the like to the operator.

In Embodiment 2, the transmission rate is changed in accordance with the confidence, but a color, a color phase, a chromatic value, a lightness value, and the like may be changed in accordance with the confidence.

### (Embodiment 3)

In Embodiment 3, a configuration will be described in which a display color set for the pancreas portion and a display color of the pancreas portion in the operative field image are averaged, and the pancreas portion is displayed by being colored with the averaged color.

Note that, the entire configuration of the laparoscope surgery assistance system, and the internal configuration of the information processing device 200, and the like are the same as those in Embodiment 1, and thus, the description thereof will be omitted.

In a case where the recognition image of the pancreas portion is obtained, the information processing device 200 according to Embodiment 3 averages the display color set in advance for the recognition image and the display color of the operative field image of the background, and displays the averaged color as the display color of the pancreas portion. For example, in a case where the display color set for the pancreas portion is set as (R1, G1, B1), and the display color of the pancreas portion in the operative field image of the background is set as (R2, G2, B2), the control unit 201 may display the pancreas portion by coloring the pancreas portion with the color of ((R1 + R2)/2, (G1 + G2)/2, (B1 + B2)/2). Alternatively, weight coefficients W1 and W2 may be introduced, and the recognized pancreas portion may be displayed by being colored with the color of (W1 × R1 + W2 × R2, W1 × G1 + W2 × G2, W1 × B1 + W2 × B2).

In Embodiment 3, the pancreas portion is colored by averaging the display color set for the pancreas portion and the display color of the pancreas portion in the operative field image, and thus, it is possible to allow the pancreas portion to blend in with the operative field image, and provide an image with a less uncomfortable feeling to the operator.

### (Embodiment 4)

In Embodiment 4, a configuration will be described in which the information processing device 200 includes a plurality of types of learning models.

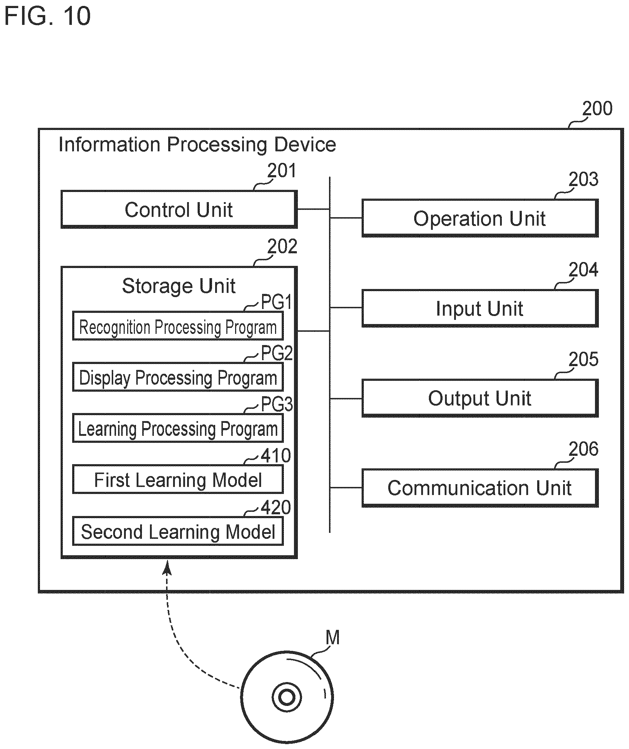

FIG. 10 is a block diagram illustrating the internal configuration of the information processing device 200 according to Embodiment 4. The information processing device 200 according to Embodiment 4 includes a first learning model 410 and a second learning model 420. The other configuration of the information processing device 200, and the entire configuration of the system including the information processing device 200 are the same as those in Embodiments 1 to 3, and thus, the description thereof will be omitted. Note that, in this embodiment, a configuration will be described in which the information processing device 200 includes two types of learning models, but three or more types of learning models may be provided.

In Embodiment 4, the first learning model 410 is a learning model to be selected in a case where the patient is first attribute (for example, a male), and the second learning model 420 is a learning model to be selected in a case where the patient is second attribute (for example, a female).

The first learning model 410 and the second learning model 420 are the same as the learning model 300 described in Embodiment 1, and a learning model for performing the image segmentation, such as SegNet, a learning model for object detection, such as YOLO, and the like are used. The first learning model 410 is generated by using a data set including a plurality of sets of operative field images shot with respect to the patient having the first attribute and ground truth data pieces obtained by selecting the pixel corresponding to the pancreas portion in the operative field image in pixel unit, as the training data. Similarly, the second learning model 420 is generated by using a data set including a plurality of sets of operative field images shot with respect to the patient having the second attribute and ground truth data pieces obtained by selecting the pixel corresponding to the pancreas portion in the operative field image in pixel unit, as the training data. The learning procedure is the same as that in Embodiment 1, and thus, the description thereof will be omitted.

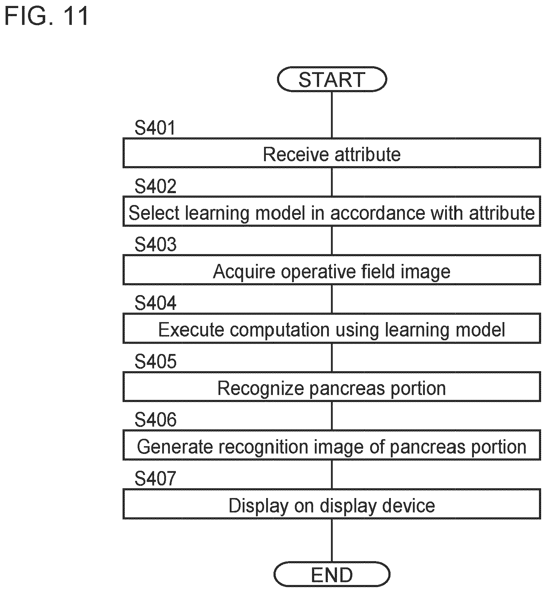

FIG. 11 is a flowchart illustrating a procedure of processing executed by the information processing device 200 according to Embodiment 4. The control unit 201 of the information processing device 200 receives information for the attribute of the patient through the operation unit 203 (step S401).

The control unit 201 selects the learning model, in accordance with the attribute of the patient received in step S401 (step S402). In a case where the attribute received in step S401 is the first attribute, the control unit 201 may select the first learning model 410, and in a case where the attribute received in step S401 is the second attribute, the control unit 201 may select the second learning model 420.

The control unit 201 executes the same procedure of steps S403 to S407 as that in Embodiment 1 to recognize the pancreas portion by using the selected learning model (the first learning model 410 or the second learning model 420), and display the recognition image of the pancreas portion on the display device 130 to be superimposed on the operative field image.

Note that, the first learning model 410 may be a learning model selected for a patient subjected to a chemotherapy, and the second learning model 420 may be a learning model selected for an obese patient. Other learning models selected in accordance with the attribute of the patient such as the age, the height, and the weight may be provided. The information processing device 200 may select a suitable learning model (for example, the first learning model 410, the second learning model 420, or the like), with reference to information such as prior information before surgery input from the outside, such as an electronic medical record, a surgical approach, the portion of the pancreas (the pancreatic uncinate process, the pancreatic head, the pancreatic body, the pancreatic tail, or the like), and the type of imaging device 11B shooting the operative field.

In addition, the first learning model and the second learning model may be learning models trained by different algorithms such that the first learning model 410, for example, is a learning model constructed by SegNet, and the second learning model 420, for example, is a learning model constructed by U-Net. In this case, the information processing device 200 may receive the selection of the learning model by the operator through the operation unit 203 or the communication unit 206.

### (Embodiment 5)

In Embodiment 5, a configuration will be described in which the optimal learning model is selected in accordance with the operative field image to be input.

As with Embodiment 4, the information processing device 200 according to Embodiment 5 includes the first learning model 410 and the second learning model 420. The first learning model 410, for example, is the learning model constructed by SegNet, and the second learning model 420, for example, is the learning model constructed by U-Net. The combination of neural networks for constructing the first learning model 410 and the second learning model 420 is not limited to the above, and any neural network may be used.

Alternatively, the first learning model 410 and the second learning model 420 may be learning models having different internal configurations. For example, the first learning model 410 and the second learning model 420 may be learning models constructed with the same neural network, but the type of layer or the number of layers, the number of nodes or a connection relationship between the nodes, and the like may be different.

In addition, the first learning model 410 and the second learning model 420 may be learning models trained by using different training data pieces. For example, the first learning model 410 may be a learning model trained by using training data including ground truth data annotated by a first specialist, and the second learning model 420 may be a learning model trained by using training data including ground truth data annotated by a second specialist different from the first specialist. In addition, the first learning model 410 may be a learning model trained by using training data including an operative field image shot in a certain health institute and annotation data (ground truth data) with respect to the operative field image, and the second learning model 420 may be a learning model trained by using training data including a operative field image shot in another health institute and annotation data (ground truth data) with respect to the operative field image.

In a case where the operative field image is input, the information processing device 200 executes computation by the first learning model 410 and computation by the second learning model 420 in the control unit 201. In order to execute such computations in parallel, the control unit 201 may include a plurality of computation units (for example, a plurality of GPUs). The control unit 201 analyzes a computation result by the first learning model 410 and a computation result by the second learning model 420, and selects the learning model (the first learning model 410 or the second learning model 420) optimal to the recognition of the pancreas portion, on the basis of an analysis result.

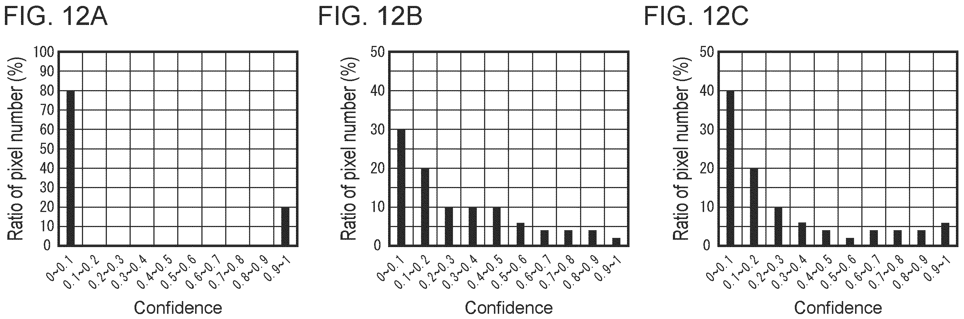

FIG. 12 is an explanatory diagram illustrating an analytical method of the computation result. A probability (a confidence) indicating whether each of the pixels corresponds to the pancreas portion is output as the computation result from each of the learning models for recognizing the pancreas portion. In a case where the pixel number is added up for each confidence, for example, a distribution as illustrated in FIG. 12A to FIG. 12C is obtained. In each graph illustrated in FIG. 12A to FIG. 12C, a horizontal axis indicates the confidence, and a vertical axis indicates the pixel number (a ratio to the entire image). Ideally, each of the pixels is classified into a case where the confidence is 1 (a probability that it is the pancreas portion is 100%) or a case where the confidence is 0 (the probability that it is the pancreas portion is 0), and thus, in a case where the distribution of the confidences is examined on the basis of the computation result obtained from an ideal learning model, a polarized distribution as illustrated in FIG. 12A is obtained.

In a case where the computation result is acquired from the first learning model 410 and the second learning model 420, the control unit 201 of the information processing device 200 adds up the pixel number for each of the confidences, and selects a learning model having a distribution close to the ideal distribution. For example, in a case where the distribution obtained from the computation result of the first learning model 410 is a distribution illustrated in FIG. 12B, and the distribution obtained from the computation result of the second learning model 420 is a distribution illustrated in FIG. 12C, the latter case is close to the ideal distribution, and thus, the control unit 201 selects the second learning model 420.

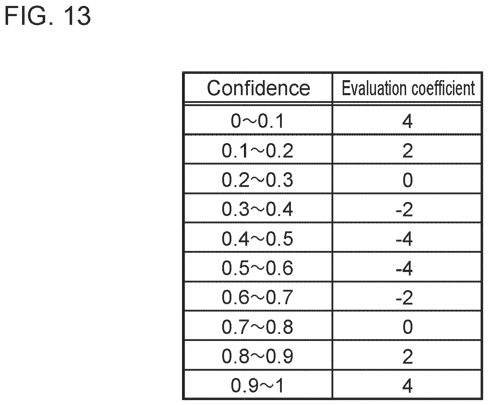

The control unit 201, for example, evaluates each distribution by using an evaluation coefficient in which an evaluation value increases as the confidence approaches 1 or 0 to determine whether the distribution is close to the ideal distribution. FIG. 13 is a diagram illustrating an example of an evaluation coefficient table. Such an evaluation coefficient table is prepared in advance in the storage unit 202. In the example of FIG. 13, the evaluation coefficient is set to have a higher value as the confidence approaches 1 or 0.

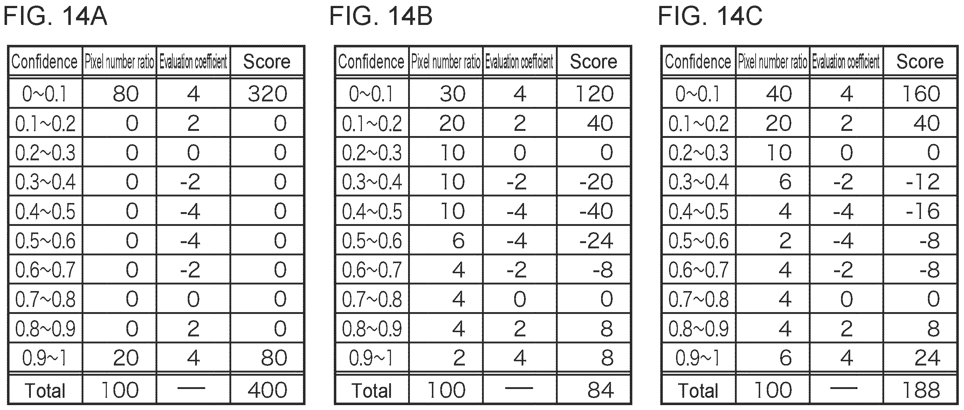

In a case where a result of adding up the pixel number for each of the confidences is obtained, the control unit 201 computes a score indicating the quality of the distribution by multiplying the result and the evaluation coefficient together. FIG. 14 is a diagram illustrating an example of a score computation result. FIG. 14A to FIG. 14C illustrate a result of computing the score for each of the distributions illustrated in FIG. 12A to FIG. 12C. A score computed from the ideal distribution is the highest score. In the case of computing the score for the distribution obtained from the computation result of the first learning model 410, the total score is 84, and in the case of computing the score for the distribution obtained from the computation result of the second learning model 420, the total score is 188. In this case, since the second learning model 420 has a higher score than the first learning model 410, the control unit 201 selects the second learning model 420, as a suitable learning model.

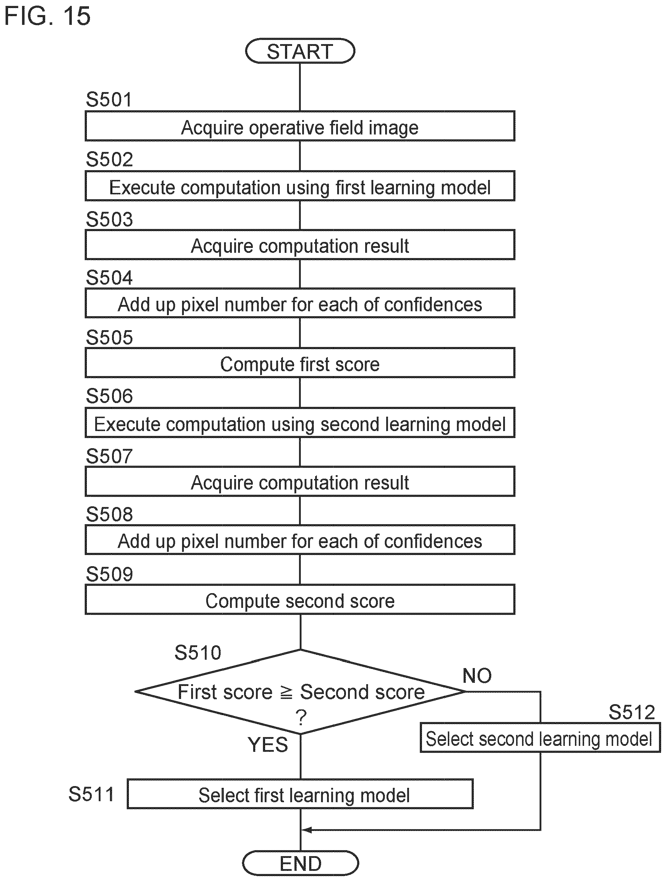

FIG. 15 is a flowchart illustrating a procedure of processing executed by the information processing device 200 according to Embodiment 5. In a case where the operative field image is acquired (step S501), the control unit 201 executes the computation by the first learning model 410 (step S502), and acquires the computation result by the first learning model 410 (step S503). The control unit 201 adds up the pixel number for each of the confidences to the first learning model 410 (step S504), and computes the score (a first score) of the distribution by multiplying each of the added pixel numbers and the evaluation coefficient together (step S505).

Similarly, the control unit 201 executes the computation by the second learning model 420 for the operative field image acquired in step S501 (step S506), and acquires the computation result by the second learning model 420 (step S507). The control unit 201 adds up the pixel number for each of the confidences to the second learning model 420 (step S508), and computes the score (a second score) of the distribution by multiplying each of the added pixel numbers and the evaluation coefficient together (step S509).

In this flowchart, for convenience, the computation (S502 to S505) for the first learning model 410 is executed, and then, the computation (S506 to S509) for the second learning model 420 is executed, but such a procedure may be reversed, or may be executed in a simultaneous and parallel manner.

The control unit 201 compares the first score with the second score, and determines whether the first score is the second score or more (step S510).

In a case where it is determined that the first score is the second score or more (S510: YES), the control unit 201 selects the first learning model 410 as a suitable learning model (step S511). Thereafter, the control unit 201 executes the recognition processing of the pancreas portion by using the selected first learning model 410.

In a case where it is determined that the first score is less than the second score (S510: NO), the control unit 201 selects the second learning model 420 as a suitable learning model (step S512). Thereafter, the control unit 201 executes the recognition processing of the pancreas portion by using the selected second learning model 420.

As described above, in Embodiment 5, it is possible to execute the recognition processing of the pancreas portion by selecting a more suitable learning model.

The information processing device 200 may execute the recognition processing of the pancreas portion using the computation result of the first learning model 410 in the foreground, and may execute the computation of the second learning model 420 in the background. The control unit 201 may evaluate the first learning model 410 and the second learning model 420 at a periodic timing, and switch the learning model used in the recognition of the pancreas portion, in accordance with an evaluation result. In addition, the control unit 201 may evaluate the first learning model 410 and the second learning model 420 at a timing when the instruction is applied from the operator or the like, and switch the learning model used in the recognition of the pancreas portion, in accordance with an evaluation result.

In Embodiment 5, a method using the evaluation coefficient has been described as an evaluation method of the first learning model 410 and the second learning model 420, but the evaluation method is not limited to the method using the evaluation coefficient, and the evaluation can be performed by using various statistical indices. For example, the control unit 201 may obtain a dispersion or a standard deviation regarding the distribution, and in a case where the dispersion or the standard deviation is high, determine that the distribution is polarized. In addition, the control unit 201 may evaluate the computation result of each model by taking the value of a 100-pixel ratio (%) as the value of the vertical axis of the graph, and obtaining the peakedness or the skewness of the graph. Further, the control unit 201 may evaluate the computation result of each model by using a mode value, a percentile, or the like.

### (Embodiment 6)

In Embodiment 6, a configuration will be described in which the pancreas portion is recognized by the first learning model 410, and other portions to be distinguished from the pancreas portion are recognized by the second learning model 420.

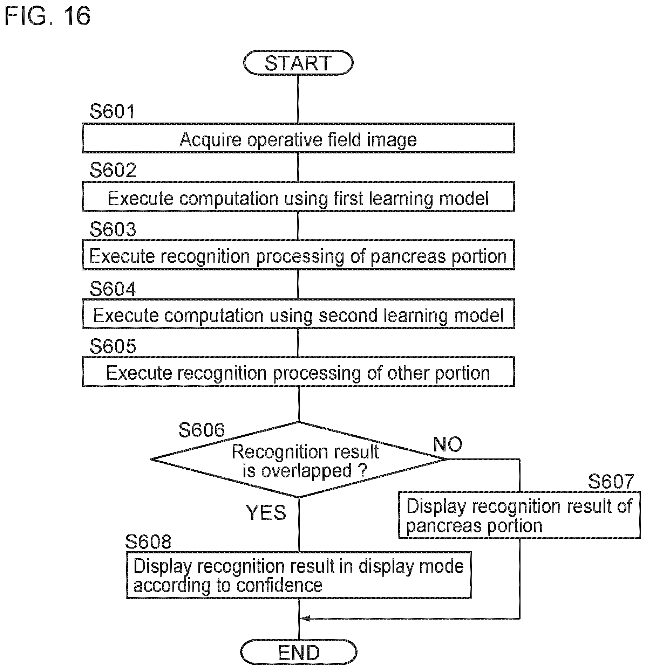

FIG. 16 is a flowchart illustrating a procedure of processing executed by the information processing device 200 according to Embodiment 6. The control unit 201 of the information processing device 200 reads out the recognition processing program PG1 and the display processing program PG2 from the storage unit 202 and executes to perform the processing, in accordance with the following procedure. In a case where the laparoscope surgery is started, the operative field image obtained by shooting the operative field with the imaging device 11B of the laparoscope 11 is continually output to the CCU 110 through the universal cord 11D. The control unit 201 of the information processing device 200 acquires the operative field image in frame unit that is output from the CCU 110 by the input unit 204 (step S601).

The control unit 201 inputs the acquired operative field image to the first learning model 410, and executes the computation by the first learning model 410 (step S602). The control unit 201 executes the recognition processing of the pancreas portion, with reference to the computation result by the first learning model 410 (step S603). The control unit 201 extracts a pixel in which the probability of the label output from the softmax layer of the first learning model 410 is the threshold value or more (for example, 60% or more), and thus, it is possible to recognize the pancreas portion included in the operative field image.

Similarly, the control unit 201 inputs the acquired operative field image to the second learning model 420, and executes the computation by the second learning model 420 (step S604). The second learning model 420 in Embodiment 6 is a learning model trained to recognize the other portion to be distinguished from the pancreas portion. The control unit 201 executes the recognition processing of the other portion to be distinguished from the pancreas portion, with reference to the computation result by the second learning model 420 (step S605). The control unit 201 extracts the pixel in which the probability of the label output from the softmax layer of the second learning model 420 is the threshold value or more (for example, 60% or more), and thus, it is possible to recognize the other portion to be distinguished from the pancreas portion, which is included in the operative field image. The control unit 201 may recognize the portion corresponding to the blood vessel, the fat, the interlobular groove, or the interlobular shadow, or may recognize organs adjacent to the pancreas, such as the stomach, the duodenum, and the spleen, by using the second learning model 420.

In the flowchart of FIG. 16, for convenience, the computation by the first learning model 410 is executed, and then, the computation by the second learning model 420 is executed, but the computation by the first learning model 410 and the computation by the second learning model 420 may be implemented in a simultaneous and parallel manner. In addition, a computation unit executing the computation by the second learning model 420 may be further provided separately from the computation unit (the control unit 201) executing the computation by the first learning model 410.

The control unit 201 determines whether the recognition result of the pancreas portion overlaps with the recognition result of the other portion (step S606). In this step, whether a specific region included in the operative field image is recognized as the pancreas portion on the one hand and recognized as the other portion on the other hand is checked. Specifically, in a case where in one pixel in the operative field image, the specific region is recognized as the pancreas portion on the one hand and recognized as the other portion on the other hand, the control unit 201 determines that the recognition results overlap with each other. In addition, it may be determined that the recognition results overlap with each other by comparing a region in the operative field image recognized as the pancreas portion with a region in the operative field image recognized as the other portion. For example, in a case where the overlap between the recognition results is a predetermined ratio or more (for example, 40% or more) at an area ratio, it may be determined that the recognition results overlap with each other, and in a case where the overlap is less than the predetermined ratio, it may be determined that the recognition results do not overlap with each other.

In a case where it is determined that the recognition results do not overlap with each other (S606: NO), the control unit 201 outputs the recognition result of the pancreas portion (step S607). Specifically, the control unit 201 displays the recognition image of the pancreas portion to be superimposed on the operative field image.

In this embodiment, in a case where it is determined that the recognition results do not overlap with each other, the recognition result of the pancreas portion is output, but the recognition result of the other portion may be output together, and the recognition image of the other portion may be also displayed on the operative field image.

In a case where it is determined that the recognition results overlap with each other (S606: YES), the control unit 201 displays the recognition image in the display mode according to the confidence (step S608). For example, in a case where the recognition result of the pancreas portion by the first learning model 410 overlaps with the recognition result of the other portion by the second learning model 420, the control unit 201 may select the recognition result with a higher confidence, and display the recognition image based on the selected recognition result.

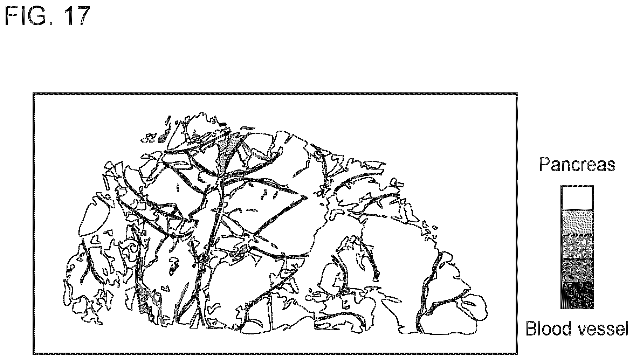

In a case where the recognition result of the pancreas portion by the first learning model 410 overlaps with the recognition result of the other portion by the second learning model 420, the control unit 201 may derive the recognition result according to the confidence, and display the recognition result in the display mode according to the confidence. FIG. 17 is a schematic view illustrating a display example of the recognition result according to the confidence. For example, in a case where as a result of recognizing a specific region included in the operative field image by the first learning model 410, the specific region is recognized as the pancreas portion with a confidence of 95%, and as a result of recognizing the same region by the second learning model 420, the region is not recognized as the other portion, the control unit 201 presents the region to the operator by coloring the region, for example, with a white color. Similarly, in a case where as a result of recognizing another region included in the operative field image by the second learning model 420, the region is recognized as the other portion with a confidence of 90%, and as a result of recognizing the same region by the first learning model 410, the region is not recognized as the pancreas portion, the control unit 201 presents the region to the operator by coloring the region, for example, with a red color (in the drawing, a black color). On the other hand, in a case where as a result of recognizing still another region included in the operative field image by the first learning model 410, the region is recognized as the pancreas portion with a confidence of 60%, and as a result of recognizing the same region by the second learning model 420, the region is recognized as the other portion with a confidence of 60%, the control unit 201 presents the region to the operator by coloring the region, for example, with an intermediate color between a white color and a red color (in the drawing, a grey color). Instead of the configuration in which the color is changed in accordance with the confidence, a configuration may be adopted in which a chromatic value, a transmission rate, or the like is changed.

### (Embodiment 7)

In Embodiment 7, a configuration will be described in which the display and the non-display of the pancreas portion are switched in accordance with the dynamic state of the operative field image.

A time lag may occur in the recognition processing of the pancreas, in accordance with a computation rate of the information processing device 200. Accordingly, for example, in the case of displaying the pancreas portion recognized by using the learning model 300 while expanding a portion including the diseased portion with the forceps 13 or while moving the energy treatment tool 12, there may be a shift between the position of the pancreas portion to be displayed and the position of the actual pancreas. Therefore, the information processing device 200 according to Embodiment 7 analyzes the dynamic state of the operative field image, and switches the display and the non-display of the pancreas portion that is the recognition result, in accordance with the dynamic state.

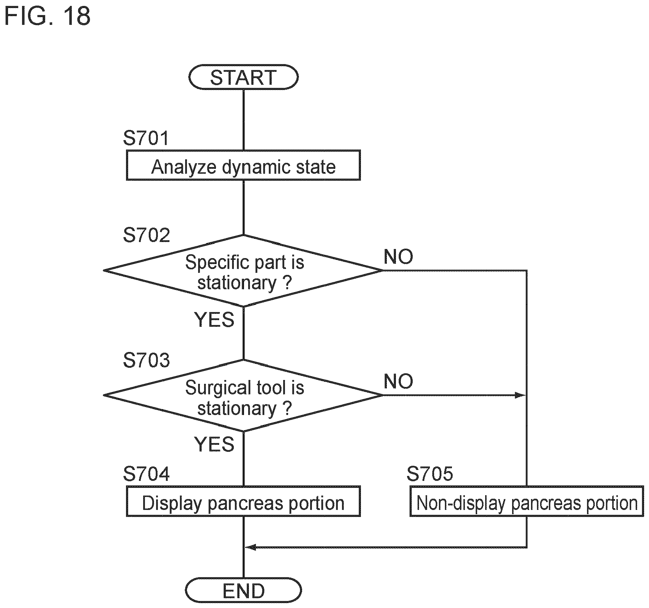

FIG. 18 is a flowchart illustrating a display switching procedure in Embodiment 7. The control unit 201 of the information processing device 200, for example, executes the following processing each time when the operative field image in frame unit is acquired by the input unit 204.

The control unit 201 of the information processing device 200 analyzes the dynamic state of the operative field by using the operative field image sequentially input by the input unit 204 (step S701). The control unit 201, for example, generates an optical flow, on the basis of a frame image of a time t-1 and a frame image of a time t, and thus, it is possible to analyze the dynamic state of the operative field. Specifically, the control unit 201 analyzes whether a specific part in the operative field image is stationary, or whether the surgical tool included in the operative field image is stationary, as the dynamic state of the operative field. Note that, the optical flow is generated by using a known method.

The control unit 201 determines whether the specific part is stationary, on the basis of an analysis result of step S701 (step S702). For example, the control unit 201 determines whether the pancreas portion is stationary. Alternatively, the control unit 201 may determine whether a region including the pancreas portion is stationary, or may determine whether a portion around the pancreas portion is stationary. Note that, in step S702, it is not necessary that the specific part is completely stationary, and it may be determined that the specific part is stationary in a case where the portion is considered to be substantially stationary (in a case where a rate or an acceleration rate computed by the optical flow is less than a setting value).

The control unit 201 determines whether the surgical tool is stationary, on the basis of the analysis result of step S701 (step S703). In step S703, it is not necessary that the surgical tool is completely stationary, and it may be determined that the surgical tool is stationary in a case where the tool is considered to be substantially stationary (in a case where a rate is less than a setting value or a case where an acceleration rate is less than a setting value).

In a case where it is determined that the specific part in the operative field image is stationary (S702: YES), and the surgical tool is stationary (S703: YES), the control unit 201 discriminably displays the recognized pancreas portion on the operative field image (step S704). Specifically, in order to discriminably display the pancreas portion recognized by using the learning model 300, the control unit 201 generates the recognition image in which a specific color is allocated to the pixel of the portion and a transmission rate is set for pixels other than the pancreas such that the background is transmissive. The control unit 201 outputs the generated recognition image to the display device 130 together with the operative field image, and displays the recognition image to be superimposed on the operative field image to display the pancreas portion.

In a case where it is determined that the specific part in the operative field image is not stationary (S702: NO) or in a case where it is determined that the surgical tool in the operative field image is not stationary (S703: NO), the control unit 201 sets non-display for the recognized pancreas portion (step S705), and ends the processing according to this flowchart. The control unit 201 may also set the transmission rate for the pixel of the recognized pancreas portion such that the background is transmissive. The control unit 201 outputs the generated recognition image to the display device 130 together with the operative field image, and displays the recognition image to be superimposed on the operative field image, and thus, it is possible to set non-display for the pancreas portion. Instead of setting non-display for the pancreas portion by changing the transmission rate, the output of the recognition image may be stopped.

Note that, in Embodiment 7, in a case where it is determined that both of the specific part and the surgical tool are stationary, the recognized pancreas portion is displayed, but in a case where it is determined that either the specific part or the surgical tool is stationary, the recognized pancreas portion may be displayed.

As described above, in Embodiment 7, in a case where the specific part and the surgical tool are stationary, the pancreas portion is displayed, and thus, it is possible to provide an image not causing an uncomfortable feeling due to a time lag to the operator.

### (Embodiment 8)

In Embodiment 8, a configuration will be described in which the display and the non-display of the pancreas portion are periodically switched.

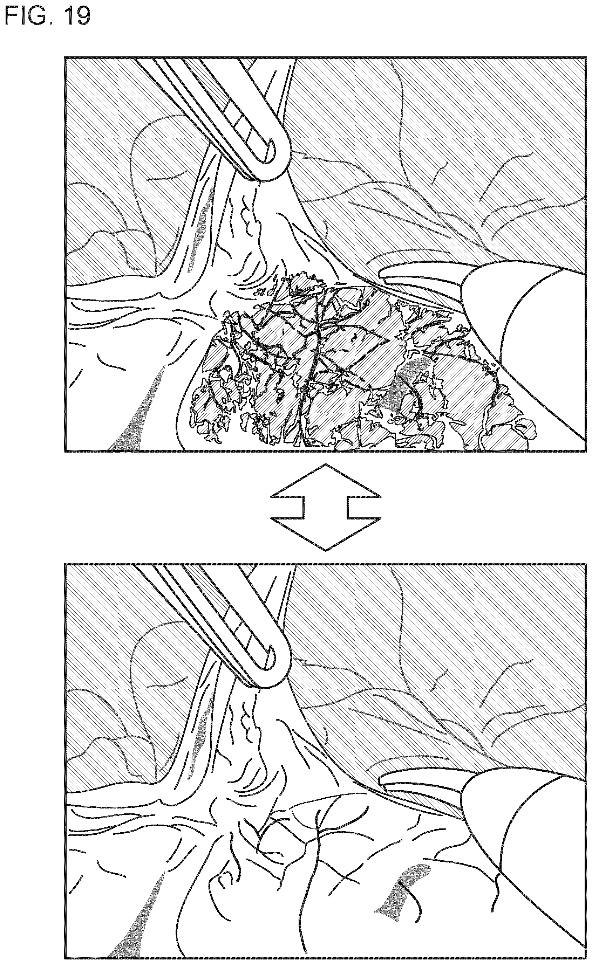

FIG. 19 is an explanatory diagram illustrating a display example in Embodiment 8. In a case where the pancreas portion is recognized by the operative field image, the information processing device 200 according to Embodiment 8 periodically switches the display of the recognized pancreas portion and the non-display of the pancreas portion. As an example, the upper diagram of FIG. 19 illustrates a state where display is set for the pancreas portion, and the lower diagram of FIG. 19 illustrates a state where non-display is set for the pancreas portion. In Embodiment 8, a display state as illustrated in the upper diagram of FIG. 19 and a non-display state as illustrated in the lower diagram of FIG. 19 are periodically switched.

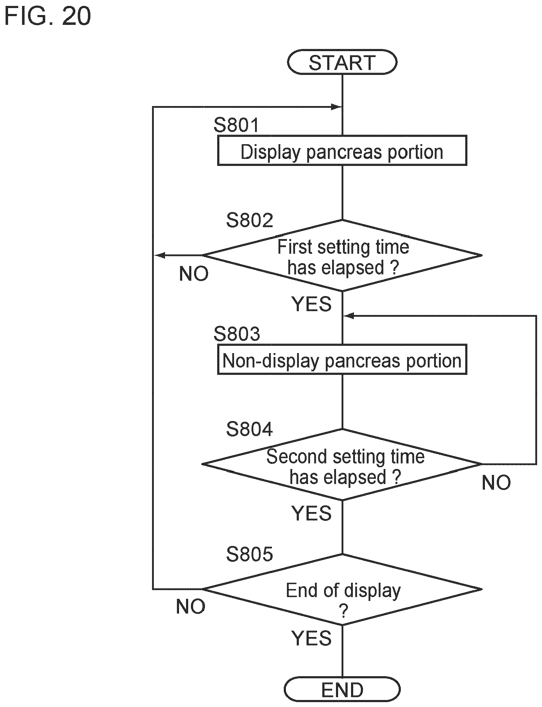

FIG. 20 is a flowchart illustrating a procedure of processing executed by the information processing device 200 according to Embodiment 8. In a case where the pancreas portion is recognized by using the learning model 300, the control unit 201 of the information processing device 200 displays the pancreas portion to be superimposed on the operative field image (step S801). The control unit 201 clocks a display time for the pancreas portion by a built-in clock, and determines whether a first setting time has elapsed (step S802). The first setting time may be a value (for example, for 2 seconds) set in advance in the device, or may be a value set by the operator through the operation unit 203. In a case where it is determined that the first setting time has not elapsed (S802: NO), the control unit 201 returns the processing to S801, and continues the display of the pancreas portion.

In a case where it is determined that the first setting time has elapsed (S802: YES), the control unit 201 sets non-display for the recognized pancreas portion (step S803). The control unit 201 may set the non-display by changing the transmission rate of the pancreas portion, or may set the non-display by stopping the output of the recognition image of the pancreas portion.

The control unit 201 clocks a time when the non-display is set for the pancreas portion by the built-in clock, and determines whether a second setting time has elapsed (step S804). The second setting time may be a value (for example, for 2 seconds) set in advance in the device, or may be a value set by the operator through the operation unit 203. Note that, the second setting time may be the same value as that of the first setting time, or may be a value different from that of the first setting time. In a case where it is determined that the second setting time has not elapsed (S804: NO), the control unit 201 returns the processing to S803, and continues the non-display of the pancreas portion.

In a case where it is determined that the second setting time has elapsed (S804: YES), the control unit 201 determines whether to end the display of the recognition result by the learning model 300 (step S805). In the case of stopping the display of the operative field image or in a case where the pancreas portion is not included in the acquired operative field image, the control unit 201 determines to end the display of the recognition result. In a case where the display is not ended (S805: NO), the control unit 201 returns the processing to step S801, and in a case where the display is ended (S805: YES), the processing according to this flowchart is ended.

As described above, in Embodiment 8, the display state and the non-display state of the pancreas portion are periodically switched, and thus, the visual assistance is performed with respect to the operator, it is possible for the operator to visually check the state of the pancreas, and safety during the surgery is improved.

Note that, in Embodiment 8, two states of the display state and the non-display state of the pancreas portion are switched, but switching from the display state to the non-display state and switching from the non-display state to the display state may be performed by stages. For example, the control unit 201 may gradually increase the transmission rate of the pancreas portion to switch from the display state to the non-display state by stages in a display period of the pancreas portion, or may gradually decrease the transmission rate of the pancreas portion to switch from the non-display state to the display state by stages in a non-display period of the pancreas portion.

### (Embodiment 9)

In Embodiment 9, a configuration will be described in which an effect is applied to the display of the pancreas portion.

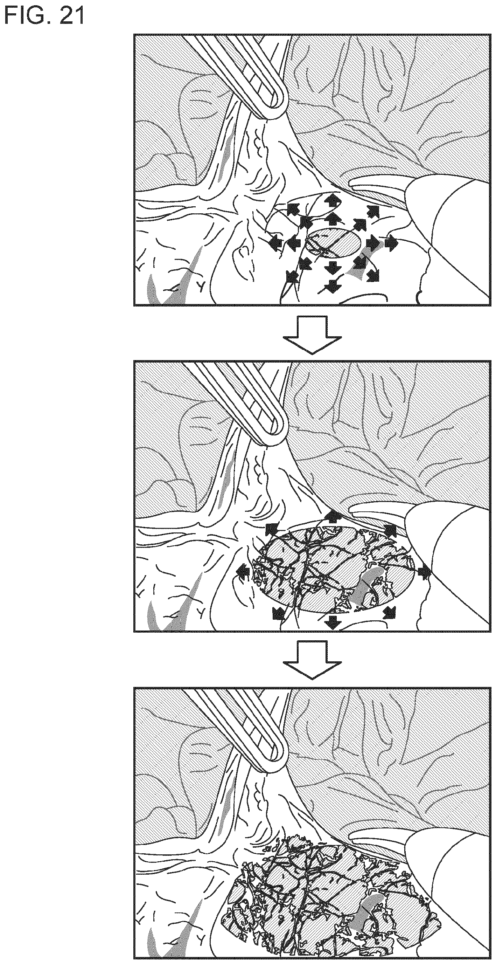

FIG. 21 is an explanatory diagram illustrating a first display example in Embodiment 9. The first display example indicates an example of applying an effect such that the display size of the pancreas portion gradually increases. In a case where the pancreas portion is recognized, the control unit 201 sets a display region (an elliptical region in the example of FIG. 21) with a size that is smaller than the original size to be displayed, and displays the pancreas portion falling within the display region. The control unit 201 sets the transmission rate of the pancreas portion within the display region to 1 (= non-transmissive), and sets the transmission rate of the pancreas portion outside the display region to 0 (= fully transmissive), and thus, it is possible to display the pancreas portion only in the display region. The control unit 201 gradually enlarges the size of the display region as time proceeds to display the entire pancreas portion that is finally recognized. The control unit 201 may repeatedly reduce and enlarge the display region. In addition, the control unit 201 may repeatedly reduce and enlarge the display region, in synchronization with the pulse of the patient.

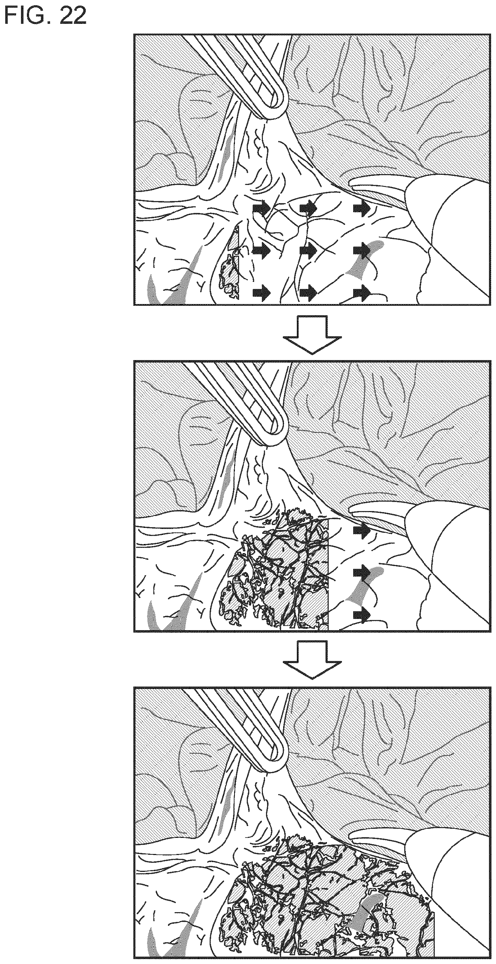

FIG. 22 is an explanatory diagram illustrating a second display example in Embodiment 9. The second display example indicates an example of applying an effect such that the pancreas portion is displayed in order from the left end. The control unit 201 sets a rectangular display region on the left end of the pancreas portion in the case of recognizing the pancreas portion, and displays the pancreas portion falling within the display region. The control unit 201 sets the transmission rate of the pancreas portion inside the display region to 1 (= non-transmissive), and sets the transmission rate of the pancreas portion outside the display region to 0 (= fully transmissive), and thus, it is possible to display the pancreas portion only in the display region. The control unit 201 enlarges the size of the display region in a right direction as time proceeds to display the entire pancreas portion that is finally recognized. The control unit 201 may repeatedly reduce and enlarge the display region. In addition, the control unit 201 may repeatedly reduce and enlarge the display region, in synchronization with the pulse of the patient. In the second display example, an effect of displaying the pancreas portion in the right direction from the left end has been described, but an effect of displaying the pancreas portion in a left direction from the right end may be applied, or an effect of displaying the pancreas portion in an upper direction (a lower direction) from the lower end (the upper end) may be applied.

In Embodiment 9, the effect is applied when displaying the pancreas portion, and thus, it is easy to visually recognize the pancreas portion, and it is possible to perform the visual assistance satisfactorily.

Note that, in Embodiment 9, the effect of gradually enlarging the display size has been described, but an effect of emphasizing the outline (the boundary) of the recognized pancreas portion may be applied, or an effect of emphasizing the outline (the boundary) of the pancreas portion, gradually transitioning the inside of the outline (the boundary) from the display state to the non-display state, and leaving only the outline (the boundary) as a residual image may be applied.

### (Embodiment 10)

In Embodiment 10, a user interface provided in the information processing device 200 will be described.

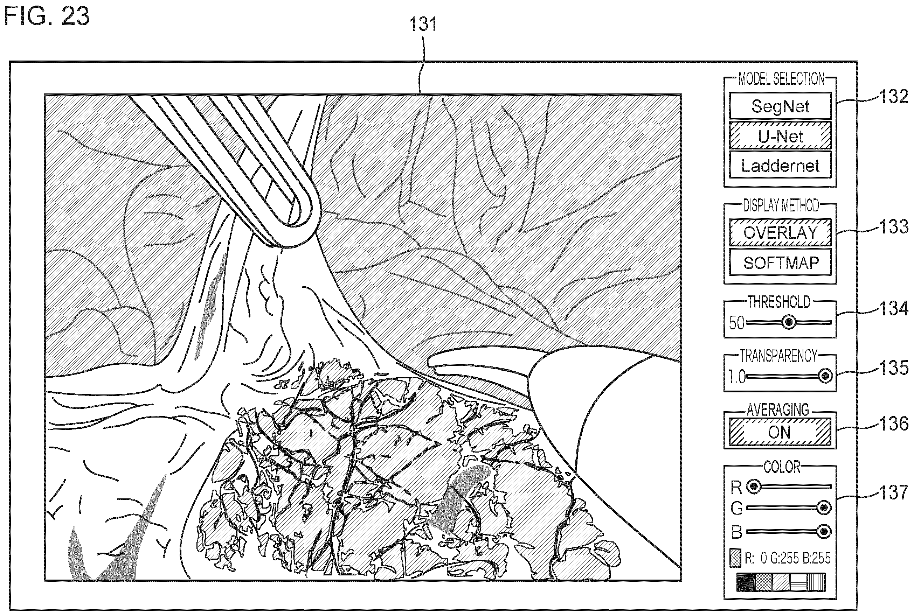

FIG. 23 is a schematic view illustrating a configuration example of the user interface provided in the information processing device 200. FIG. 23 illustrates an example in which a display region 131 for displaying the recognition image of the pancreas portion, and a user interface for controlling the display mode of the recognition image are arranged in parallel. The user interface illustrated in FIG. 23 includes a model selection unit 132, a display method selection unit 133, a threshold value setting unit 134, a transmission rate setting unit 135, an averaging instruction unit 136, and a display color selection unit 137. Various buttons or sliders provided in the user interface are manipulated by the operation unit 203 provided in the information processing device 200.

The model selection unit 132 includes a selection button for selecting the neural network for constructing the learning model 300. The example of FIG. 23 illustrates a state where "U-Net" is selected. In addition, the model selection unit 132 may include a selection button for receiving any one selection of the first learning model 410 and the second learning model 420 described in Embodiment 6. Further, the model selection unit 132 may highlight-display a recommended model, in accordance with the attribute of the patient, the prior information before surgery, the surgical approach, the portion of the pancreas, the type of imaging device shooting the operative field, or the like.

The display method selection unit 133 includes a selection button for receiving any one selection of overlay display and software map display. As described in Embodiment 2, the overlay display is a display method for uniformly displaying the pancreas portion with the same color, and the software map display is a display method for changing the transmission rate, in accordance with the confidence. The example of FIG. 23 illustrates a state where "overlay" display is selected.

The threshold value setting unit 134 includes a slider for setting a threshold value for determining whether an attention pixel corresponds to the pancreas. In a case where the slider slides to the left side, the threshold value decreases (it is likely to be recognized as the pancreas), and in a case where the slider slides to the right side, the threshold value increases (it is less likely to be recognized as the pancreas).

The transmission rate setting unit 135 includes a slider for changing the transmission rate of the recognition image. In a case where the slider slides to the left side, the transmission rate decreases, and in a case where the slider slides to the right side, the transmission rate increases.

The averaging instruction unit 136 includes an instruction button for turning on or off the averaging of the display colors. In a case where the averaging of the display colors is turned on, the control unit 201 averages the display color set for the recognition image (the pancreas portion) and the display color set for the operative field image of the background, and displays the averaged color as the display color of the pancreas portion. For example, in a case where the display color set for the pancreas portion is set as (R1, G1, B1), and the display color of the pancreas portion in the operative field image of the background is set as (R2, G2, B2), the control unit 201 may display the pancreas portion by coloring the pancreas portion with the color of ((R1 + R2)/2, (G1 + G2)/2, (B1 + B2)/2). Alternatively, the weight coefficients W1 and W2 may be introduced, and the recognized pancreas portion may be displayed by being colored with the color of (W1 × R1 + W2 × R2, W1 × G1 + W2 × G2, W1 × B 1 + W2 × B2).

The display color selection unit 137 includes a slider and a color palette for changing the display color of the pancreas portion. The display color selection unit 137 may set a color designated by the slider as the display color of the pancreas portion, or may set a color selected by the color palette as the display color of the pancreas portion. In addition, the display color selection unit 137 may include a default button for returning the display color changed by the user to a default display color (for example, a white color).

In a case where a change instruction for the display mode is received through the model selection unit 132, the display method selection unit 133, the threshold value setting unit 134, the transmission rate setting unit 135, the averaging instruction unit 136, and the display color selection unit 137, the control unit 201 of the information processing device 200 may change the display mode of the recognition image of the pancreas portion that is displayed in the display region 131, in accordance with the change instruction.

In the example of FIG. 23, the user interface includes the model selection unit 132, the display method selection unit 133, the threshold value setting unit 134, the transmission rate setting unit 135, the averaging instruction unit 136, and the display color selection unit 137, but the user interface for controlling the display mode of the recognition image is not limited thereto. For example, as the user interface, a selection unit for receiving the availability of inference by the learning model 300 (or the learning models 410 and 420) may be provided. Further, as the user interface, a setting unit for setting an inference start time may be provided. In addition, a setting unit for setting the first setting time and the second setting time described in Embodiment 8 may be provided.

In addition, in a case where the change of the display mode is received through the user interface as illustrated in FIG. 23, and a change occurs from default setting, the control unit 201 may notify the operator that the display mode is changed, at a suitable timing. For example, the control unit 201 may compare the default setting value of the display mode with the current setting value of the display mode when activating the information processing device 200 or when starting the surgery, and in a case where there is a difference between the setting values, display the difference on the display device 130, or notify the difference to a mobile terminal carried by the operator.

The embodiments disclosed herein are illustrative in all respects and should not be considered restrictive. In addition, the present invention is not limited to each of the embodiments described above, and can be configured by combining a plurality of embodiments or by combining a plurality of configurations in different embodiments. The scope of the present invention is indicated by the claims, and it is intended that all modifications within the meaning and range equivalent to the claims are included.

### [Description of Reference Numerals]

- 10: Chest tube
- 11: Laparoscope
- 12: Energy treatment tool
- 13: Forceps
- 110: Camera control unit (CCU)
- 120: Light source device
- 130: Display device
- 140: Video recording device
- 200: Information processing device
- 201: Control unit
- 202: Storage unit
- 203: Operation unit
- 204: Input unit
- 205: Output unit
- 206: Communication unit
- 300: Learning model
- PG1: Recognition processing program
- PG2: Display processing program
- PG3: Learning processing program

## Claims

1. A computer program causing a computer to execute processing of:
acquiring an operative field image obtained by shooting an operative field of a scope-assisted surgery; and
recognizing a pancreas portion in the acquired operative field image by inputting the acquired operative field image to a learning model trained to output information relevant to the pancreas portion included in the operative field image in accordance with input of the operative field image.

2. The computer program according to claim 1 causing the computer to further execute processing of:
recognizing the pancreas portion excluding a portion corresponding to a blood vessel, fat, an interlobular groove, or an interlobular shadow appearing on a surface of a pancreas from a region set by an outer edge of the pancreas, on the basis of the information output from the learning model; and
displaying the pancreas portion recognized by excluding the portion on the operative field image.

3. The computer program according to claim 1 or 2 causing the computer to further execute processing of:
changing a display mode of the pancreas portion, in accordance with a confidence of a recognition result of the pancreas portion.

4. The computer program according to claim 1 or 2 causing the computer to further execute processing of:
averaging a display color set for the pancreas portion and a display color of the pancreas portion in the operative field image; and
coloring and displaying the recognized pancreas portion with the averaged color.

5. The computer program according to any one of claims 1 to 4,
wherein the learning model includes a plurality of types of learning models for recognizing the pancreas portion, and
the program causes the computer to further execute processing of
selecting one learning model from the plurality of types of learning models, in accordance with attribute of a patient, prior information before surgery, a surgical approach, a part of the pancreas, or a type of imaging device shooting the operative field.

6. The computer program according to any one of claims 1 to 4,
wherein the learning model includes a plurality of types of learning models for recognizing the pancreas portion, and
the program causes the computer to further execute processing of:
evaluating each of the learning models, on the basis of information output from each of the learning models when the operative field image is input; and
recognizing the pancreas portion included in the operative field image by using a learning model selected on the basis of an evaluation result.

7. The computer program according to any one of claims 1 to 6 causing the computer to further execute processing of
switching display and non-display of the pancreas portion, in accordance with a dynamic state of a specific part in the operative field image.

8. The computer program according to any one of claims 1 to 7 causing the computer to further execute processing of
switching the display and the non-display of the pancreas portion, in accordance with a dynamic state of a surgical tool included in the operative field image.

9. The computer program according to any one of claims 1 to 6 causing the computer to further execute processing of
periodically switching display and non-display of the pancreas portion.

10. The computer program according to any one of claims 1 to 9 causing the computer to further execute processing of
applying a predetermined effect to the display of the pancreas portion.

11. A computer program causing a computer to execute processing of:
acquiring an operative field image obtained by shooting an operative field of a scope-assisted surgery;
recognizing a pancreas portion and the other portion in the acquired operative field image by inputting the acquired operative field image to a first learning model and a second learning model separately, where the first learning model being trained to output information relevant to the pancreas portion included in the operative field image, and the second learning model being trained to output information relevant to the other portion to be distinguished from the pancreas portion included in the operative field image, in accordance with input of the operative field image;
setting a display mode of the pancreas portion and the other portion on the operative field image, in accordance with a level of a confidence of a recognition result by the first learning model and a confidence of a recognition result by the second learning model; and
displaying the pancreas portion and the other portion on the operative field image in the set display mode.

12. The computer program according to claim 11 causing the computer to further execute processing of
adopting a recognition result with a higher confidence to selectively display either the pancreas portion or the other portion when a region recognized as the pancreas portion by using the first learning model overlaps with a region recognized as the other portion by using the second learning model.

13. The computer program according to claim 11 causing the computer to further execute processing of
mixing display colors, in accordance with each of the confidences, when a region recognized as the pancreas portion by using the first learning model overlaps with a region recognized as the other portion by using the second learning model, to color and display the region with the mixed display color.

14. A method for generating a learning model causing a computer to execute processing of:
acquiring training data including an operative field image obtained by shooting an operative field of a scope-assisted surgery, and ground truth data indicating a pancreas portion in the operative field image; and
generating a learning model for outputting information relevant to the pancreas portion in accordance with input of the operative field image on the basis of a set of acquired training data pieces.

15. An information processing device, comprising:
an acquisition unit that acquiring an operative field image obtained by shooting an operative field of a scope-assisted surgery;
a recognition unit recognizing a pancreas portion in the acquired operative field image by inputting the acquired operative field image to a learning model trained to output information relevant to the pancreas portion included in the operative field image in accordance with input of the operative field image; and
an output unit outputting information based on a recognition result by the recognition unit.
